(19) Europäisches Patentamt / European Patent Office / Office européen des brevets

(11) **EP 1 604 698 B1**

(12) **EUROPEAN PATENT SPECIFICATION**

(45) Date of publication and mention
of the grant of the patent:
**12.09.2018  Bulletin 2018/37**

(51) Int Cl.:
***A61M 1/16*** *(2006.01)*

(21) Application number: **05011504.7**

(22) Date of filing: **27.05.2005**

(54) **Method for conductivity calculation in a treatment fluid in apparatuses for the blood treatment**

Methode für Berechnungen auf der Basis der Leitfähigkeit einer Behandlungsflüssigkeit in einem Gerät zur Behandlung von Blut

Methode de calcul fondée sur la conductivité d'un fluide de traitement dans des appareils de traitement du sang

(84) Designated Contracting States:
**AT BE BG CH CY CZ DE DK EE ES FI FR GB GR HU IE IS IT LI LT LU MC NL PL PT RO SE SI SK TR**

(30) Priority: **08.06.2004  US 578120 P**

(43) Date of publication of application:
**14.12.2005  Bulletin 2005/50**

(73) Proprietor: **Gambro Lundia AB**
**22010 Lund (SE)**

(72) Inventors:
• **Jansson, Olof**
**SE-23538 Vellinge (SE)**
• **Sternby, Jan Peter**
**SE-22652 Lund (SE)**
• **Persson, Roland**
**SE-21611 Limhamn (SE)**

(74) Representative: **Ponzellini, Gianmarco et al**
**PGA S.P.A., Milano**
**Succursale di Lugano**
**Viale Castagnola, 21c**
**6900 Lugano (CH)**

(56) References cited:
**WO-A1-03/066135     US-A1- 2001 004 523**

## Description

### Background of Invention

The present invention relates to a blood treatment machine carrying out a method for conductivity calculation in a treatment fluid upstream and downstream from a filtration unit.

It is known in the art to use conductivity measures for determination of parameters indicative of the filter efficiency during treatment, i.e. clearance or dialysance, and for determination of patient parameters, such as fistula flow.

EP 547025 shows a first method for determining clearance starting from a perturbation of the conductivity of the upstream dialysis liquid which creates corresponding response in the conductivity of the liquid downstream the dialysis unit.

Measures of the conductivity allow determination of downstream response and calculation of the clearance.

It is also known from EP 658352 an alternative method to calculate conductivity values for clearance calculation by a short time perturbation. It is also known from document WO03/066135A1 a method to determine fistula flow by making a step like perturbation in the upstream conductivity and reversing the lines in the extracorporeal circuit during the step perturbation.

By measuring the conductivities in the spent dialysate across the flow reversal, it is possible to arrive at fistula flow determination.

### Summary of Invention

While the fistula flow calculation according to the above methods is quite acceptable, it would be highly desirable to increase accuracy trying to reduce the time of the step like perturbation. More in general it is a goal of the invention to devise a method for conductivity determination in the spent dialysate upon a perturbation in the upstream liquid, increasing the accuracy, while keeping an acceptable measurement time.

It is also an aim of the present invention to render conductivity detections as much as possible independent from the step size, from the operating conditions and from the presence of undesired disturbances or noises.

These and other aims besides which shall be made clearer in the course of the following description, are substantially attained by a method for determining the conductivity of a treatment fluid downstream from a filtration unit in blood processing machines, as described in the accompanying claims.

### Brief Description of Drawings

Further features and advantages will become more readily apparent from the detailed description of a preferred, but not exclusive, embodiment of a process for determining the treatment fluid conductivity according to the invention.

Such description shall be made hereafter with reference to the accompanying drawings, provided purely by way of non-limiting indication:

- figure 1 shows the conductivity curves upstream and downstream from the filtration unit in their time development;
- figure 2 shows a computer model generated curve of the dialysate outlet conductivity (Cdo);
- figure 3 shows real data dialysate inlet conductivity (Cdi);
- figure 4 shows a representation of the dialysate outlet conductivity curve when there is a big difference between the set conductivity and the patient conductivity;
- figure 5 shows a dialysate outlet conductivity curve part before the induced conductivity change;
- figure 6 shows a computer model representation of function unit_f;
- figure 7 shows a computer model representation of functions f_norm and f_norm_est;
- figure 8 shows a schematic representation of the dialysate inlet and outlet conductivity curve;
- figure 9 shows the effect of the flow reversal in the dialysate outlet conductivity as a small step;
- figure 10a shows a representation of function unit_$f_{acc}$;
- figure 10b shows the area associated to function unit_$f_{acc}$;
- figure 11 again shows the inlet and outlet conductivity curves and relevant steps used for calculating access flow;
- figure 12 shows two different line fits made to the Cdo curve.

### Detailed Description

The invention is defined in the appended claim 1. Dependent claims 2-47 define further preferred aspects of the invention.

The present invention relates to a peculiar technic for determining the conductivity of a treatment fluid downstream from a filtration unit in blood processing machines and also describes one technic of how clearance and access flow can be calculated.

The method is based on studying the dialysate outlet conductivity response that follows upon rising the dialysis solution conductivity and shifting the blood flow connections to the patient.

The response of a dialysis solution conductivity step is dependent on many factors.

It is therefore difficult to extract the asymptotic components needed for calculation of the clearance.

Even more so is this true for the access flow step, since the change in dialysate outlet conductivity, due to the reversal of the blood flow direction, is really small. The algorithm described below has shown to be the one that gives the best results for both clinical data as well as data generated by a computer model. The various variables used in the specification and in the claims later on are below defined in the table 1.

When relevant, the time interval from which data shall be extracted and processed for calculating the variable is given.

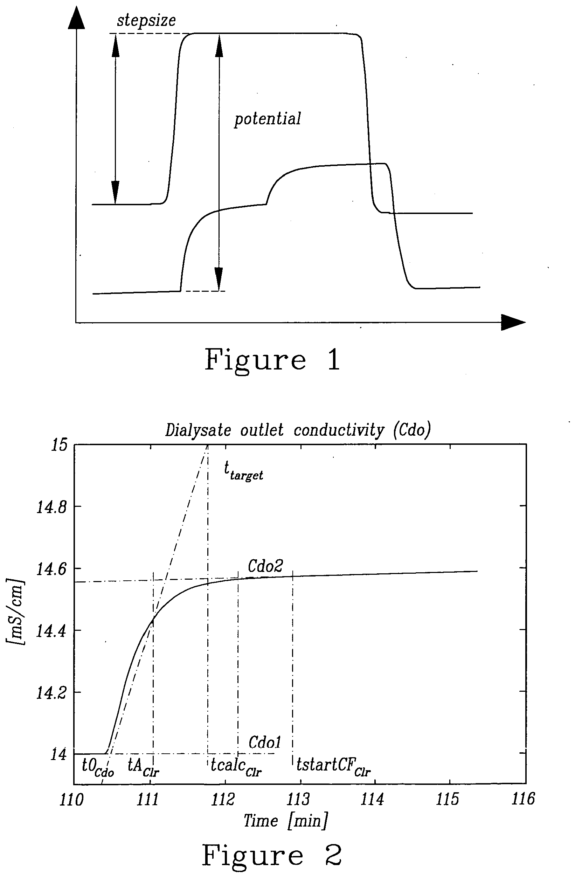

Some of the definition are also illustrated in figure 1, figure 2 and figure 9 as annexed.

TABLE 1

| Variable | Description | Data interval [minutes] | |
|---|---|---|---|
| | | Start point | End point |
| Ke | Clearance (effective) | | |
| Cdi | Dialysis solution conductivity (dialyser inlet) | | |
| Cdi1 = $Cdi_{pre,mean}$ | Inlet pre step conductivity needed for the clearance calculation. | $t0Cdi - (3 + UF_{cal}\_time)$ | $t0Cdi - UF_{Cal}\_time$ |
| Cdi2 = $Cdi_{step,mean}$ | Inlet step conductivity needed for the clearance calculation. | $t0Cdi + 3$ | $tfCdi - 1$ |
| $Cdi_{step,set}$ | The set value of Cdi during the condstep. | | |
| Cdo | Dialysate conductivity (dialyser outlet) | | |
| $Cdo_{corr}$ | Adjusted Cdo data. | $t0_{cdi} + 0.5$ | $tf_{cdi} - 0.5$ |

| Variable | Description | Data interval | |
|---|---|---|---|
| | | Start point | End point |
| Cdo1 | Outlet pre step conductivity needed for the clearance calculation. | | |
| Cdo2 | Outlet step conductivity needed for the clearance calculation. | | |
| $Cdo2_{est}$ | An estimation of Cdo2 taken at $t0_{Cdo}$. Used for adjusting data. | | |
| $Cdop_{re,mean}$ | Outlet pre step conductivity mean value | $t0Cdo - (3 + UF_{Cal}\_time + Cdo\_Cdi\_del ay)$ | $t0Cdo - (UF_{cal}\_time + Cdo\_Cdi\_delay)$ |
| Cdon | Outlet pre reversal conductivity needed for the access flow calculation. | | |
| Cdor | Outlet post reversal conductivity needed for the access flow calculation. | | |

(continued)

| Variable | Description | Data interval | |
|---|---|---|---|
| | | **Start point** | **End point** |
| $tO_{Cdi}$ | Calculated estimated time of when Cdi starts to raise. | | |
| | The area calculation, uses data between | 0.5 minutes before end of UF calibration. | Point when machine takes the Cdi down. |
| | The line fit is done on area data | 0.5 minutes after end of UF calibration | 1 minute prior to the end of the Cdi pulse |
| $tf_{Cdi}$ | Time when Cdi starts to drop back to normal. | | |
| $t0_{Clr}$ | Calculated estimated time of when Cdo starts to raise. | | |
| $UF_{Cal}\_time$ | The UF calibration takes some 45 seconds and is done prior to the raise in Cdi. Data during this period should not be used. Time set with a 15 seconds margin to 1 minute. | | |
| Cdo_Cdi_delay | How much Cdo is delayed ($t0_{Cdo}$- $t0_{Cdi}$) | | |
| $tCalc_{clr}$ | Time at which the conductivities Cdo1 and Cdo2 are taken | | |
| $tCalc_{acc}$ | Time at which the conductivities Cdon and Cdor are taken | | |

The following calculation method is particularly adapted for blood-treatment apparatuses having at least a filtration unit with a first compartment for the circulation of blood and a second compartment for the circulation of the treatment fluid; the first and second compartments are separated by interposition of at least a semi-permeable membrane known in the art.

The machine also comprises means for changing the conductivity of the treatment fluid upstream from the filtration unit such as a sodium reservoir and a controlled pump capable of injecting predetermined quantities of conductive fluid in the dialysate fluid up the filtration unit.

Obviously the treatment machine also comprises at least a first and a second sensor for measuring, respectively, the conductivity of the process fluid upstream and downstream from the unit.

A control unit governs said devices in order to change the conductivity of the process fluid and is able to receive the conductivity signals from the aforementioned first and second sensors allowing the calculation of the conductivity of the fluid as hereafter explained.

From a general point of view, after creating a flow of treatment fluid through the second compartment of the filtration unit, a change in the conductivity of the treatment fluid at the inlet of the filtration unit is imposed for a predetermined time interval.

The step in the Cdi curve is clearly shown in figure 1.

Such a step cause thereby an induced conductivity change in the fluid at the outlet of said filtration unit (see again figure 1-CdO curve).

After a predetermined time interval following the step in the inlet conductivity the blood flow to the fistula is reversed causing recirculation in the fistula and the consequent change in the conductivity curve downstream from the filtration unit (see the second step in the Cd0 curve-figure 1).

The method allows firstly to determine the conductivity value Cdo2 of the process fluid downstream from the filtration unit after the induced conductivity change used for clearance calculation and then allows to calculate also access flow.

Clearance is calculated by studying the first part of the step response curve.

Figure 2 shows a computer model generated curve of that part. The conductivity change imposed in the inlet fluid is a known change in value remaining constant over time, said change particularly being a positive change in conductivity (an increase in conductivity). The size of the step in the inlet conductivity is 1 [mS/cm] (from 14 to 15 [mS/cm]). The time points illustrated in the figure will be elucidated in the text below.

The measurement procedure is activated by the operator. Thereafter no changes to the treatment parameters are allowed in order to create stable conditions for the measurement. In a period of three minutes before the known Ultra filtration (UF) calibration, Cdo and Cdi-data are being collected. These data are necessary for the estimation of Cdo1 and Cdi1.

In connection to the UF-calibration, in fact at the end of it, a step in Cdi is initiated. After about 1 minute the response in Cdo is beginning show. After an additional of 5 minutes the operator is prompted to reverse the blood flow direction to the patient. Should the operator not have reversed the flow within 2 minutes, the measurement should be aborted.

From the time when having reversed the blood flow direction; it again takes some 1 minute until the effects show in the Cdo-curve. Just before the effects start to show we have reached as far in the measurement as to plot the curve that is seen in Figure 2. From this part of the curve, the effective clearance ($K_e$) can be calculated. The steps needed to obtain it, are described in the following part.

Fluctuations in Cdi create fluctuations in Cdo. Since we want to study the effects in Cdo of a raise in Cdi it would have been optimal if Cdi had been constant. Of course Cdi is not constant, but one way of "making it appear constant", is to compute the deviation that Cdi does from its mean value over the pulse, and then adjust the Cdo in proportion to it. Through this, we will expect a Cdo-curve close to the one we would have got if the response in Cdi had been equal to the actual pulse mean value.

To be able to perform this adjustment the two curves must be made synchronous. We therefore have to find the starting point of the curves and move one of them to the starting point of the other.

The synchronization process then allows to compare the conductivity curves upstream and downstream from the filtration unit after they have been synchronized in order thereby to determine one or more downstream conductivity values.

Finding the start of the Cdi pulse, is quite straightforward. It is done by assessing how the area under the step develops (should be an almost straight line), make a line fit to the pulse area "line" and see at what time it has its zero value ($tA_{Cdi}$). Since the Cdi curve goes up quite steeply, this time point corresponds to the sought starting point of the Cdi-curve ($t0_{Cdi}$).

In other words the characteristic time ($t0_{Cdi}$) of the upstream conductivity curve is calculated by estimating an area defining below the inlet conductivity curve; such a characteristic time is coincident with the instant at which the area under the curve takes on an average value greater than a predetermined threshold. The point in time of when Cdi goes back to normal, $tf_{Cdi}$, is also needed in the calculations later on. It could be found using the same technique as when finding $t0_{Cdi}$.

$tf_{Cdi}$ is only used for referring of other time points. We can therefore equally well use the time when the machine goes back to the set value it had before the step.

The determination of the characteristic time $t0_{Cdo}$ of the conductivity curve downstream from the filtration unit comprises a step consisting in a preliminary estimation of the value of the characteristic time and the preliminary estimate is subsequently corrected.

When finding $t0_{Cdo}$ we basically fit a curve to the initial data of the Cdo step, and where this curve intersects with $Cdo_{pre}$ we find $t0_{Cdo}$.

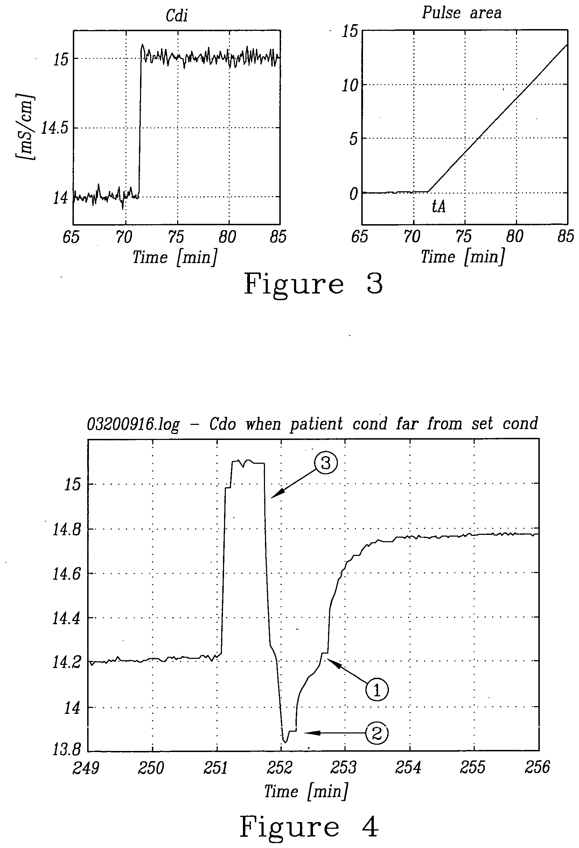

figure 4 shows what the Cdo pulse may look like when there is a big difference between the set conductivity and the patient conductivity. The point $t0_{Cdo}$ is located somewhere at 1 but could easily be pointed out to lie at 2 if data are not carefully handled.

Prior to 3 there has been a UF calibration. As can be seen, the UF calibration will result in a "bump". The bump might be quite big if, as in this case, there is a big difference between the patient conductivity and the set conductivity. What is shown here is quite extreme, but it shows an important case.

We know that the raise in conductivity is not made until the UF calibration is finished. This occurs when the bump is on its way downwards, i.e. at 3. Data prior to this point in time shall therefore not be used. We also know that it will take some minute before the raise in Cdi will start to show in Cdo. Therefore we can actually exclude another half a minute of data after the end of the UF calibration. We do not exclude the full 1 minute since we need to have some margin.

After any UF calibration there is always a "recoil effect", before Cdo returns to the course it had before the UF calibration. This means that we have some data, between 3 and 1 that should not be used either. If we do, we will, in the case described by figure 4, end up finding $t0_{Cdo}$ at 2. We must therefore confine the data to fulfill the condition Cdo > $Cdo_{pre}$ if the step is made upwards and Cdo < $Cdo_{pre}$ if the step is made downwards.

In the case of when the patient conductivity is larger than the set conductivity, the "bump" will go downwards and the recoil effect will accordingly go upwards. This means that the condition above is not enough. In this situation, when having followed the steps above, we would get Cdo-data that has a minimum at 1 (figure 4). Data prior to 1 will, if the bump is big, cause problems in the subsequent calculation of $t0_{Cdo}$. Therefore we need to exclude data prior to this point.

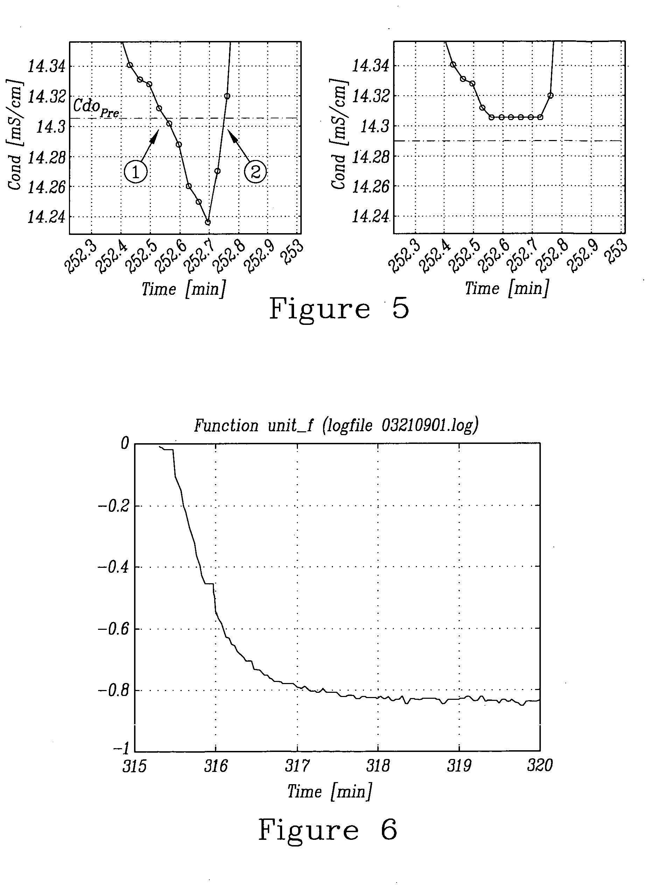

By excluding these data we have almost solved the problem. We are however not there yet, since we might in fact get the minimum at the "wrong end". Figure 5 (left) shows a situation where we have cut out a piece of the Cdo curve around the minimum. If the curve, after the recoil, passes $Cdo_{pre}$ a second time, the minimum might lie at 1 or 2 depending on how the sampling has been done. In the chosen example, the minimum will be found at 1, which is not what we want. We want 2.

We must therefore first check if there are any points lying below (or above if the step goes downwards) $Cdo_{pre}$. If so, we check which of them that has the latest time stamp. That one gives us the point we are looking for.

Furthermore, it can happen that all data are above $Cdo_{pre}$ as in Figure 5 (right). Several "minimum values" might be found, e.g. if the same value has been recorded. Again the one with the latest time stamp gives us the point we are looking for.

The case described by figure 5 (left) could probably be excluded if one always performs the step according to the difference between Cdi and Cdo prior to the step. I.e. if Cdo < Cdi the step is done upwards. Downwards if Cdo > Cdi. However, before performing a step downwards one must think about what that might do to the patient. Lowering the sodium is considered "dangerous".

The data that now remains represents the step response and the very first data point of these data could, probably in most cases, with good enough result, be used as $t0_{Cdo}$.

In other words the preliminary estimation of the value of the characteristic time of the conductivity curve downstream from the filtration unit is made by determining the average conductivity at the outlet of the filtration unit $Cdo_{pre,mean}$ prior to the effects of the change in conductivity (the determination is made on the basis of an average, i.e. arithmetic mean, of the measured conductivity values Cdo prior to the effects of the change).

Subsequently the measured conductivity values are compared with previously determined average outlet conductivity value $Cdo_{pre,mean}$ and then the instant which measured the conductivity values Cdo appear constantly greater than the previously calculated average outlet conductivity value $Cdo_{pre,mean}$ is estimated. As above stated where the measured conductivity values Cdo exceed the average outlet conductivity value $Cdo_{pre,mean}$ a number of times, the preliminary estimate of the characteristic time $t0_{Cdo}$ of the conductivity curve downstream from the filtration unit, is the instant of the first condition in which the measured conductivity values Cdo are greater than the average outlet conductivity value $Cdo_{pre,mean}$.

We shall however undertake some "additional" steps to find an even better $t0_{Cdo}$ and therefore the step of correcting the preliminary estimate of the characteristic time may be performed.

We start by creating the natural logarithm function $f = \ln(\text{sign}^*(Cdi_{step,set} - Cdo))$. The values of Cdo to use, are the ones starting at $t0_{Cdo}$ and extending up to the point in time when the reversion of the flow direction to the neddles is done. Cdi is the step set value, $Cdi_{step,set}$, and not its actual values. The function f is scaled, by "potential", so that it corresponds to a unit step. We call the new function unit_f (unit_f = $\ln(\text{sign} * (Cdi_{step,set} - Cdo)/\text{potential})$). The reason for scaling it is of course that the calculations should be the same independently of the step size or how the patient conductivity relates to the set conductivity. figure 6 shows an example of unit_f.

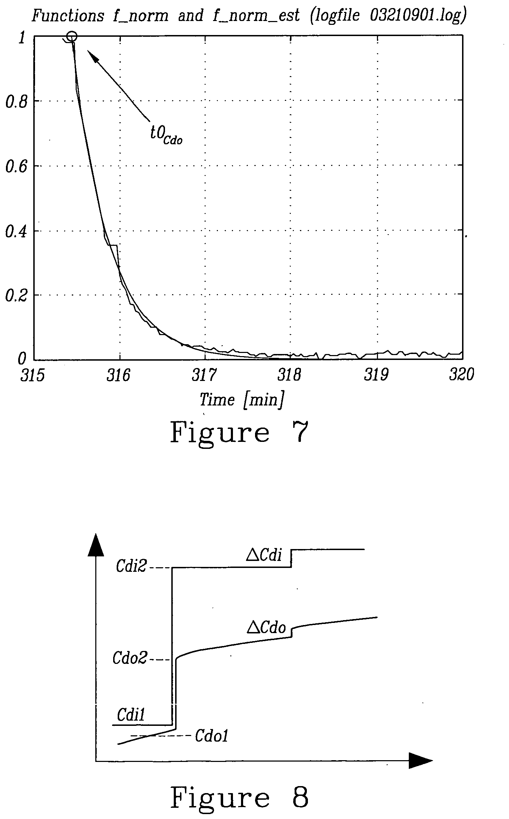

We then make a least mean square line fit to function f between t = (tRev - 1.5 minutes) and t = tRev. This range represents a part of the curve where the transients have died out. The estimation is called $Cdo\_1n_{clr}$ ($Cdo\_1n_{clr} = Cdi_{set,step} - \text{sign}^*\exp(c_{clr}(1)^*t + c_{clr}(2))$, $c_{clr}(1)$ and $c_{clr}(2)$ being the coefficients from the fit). We now construct a function $f2_{clr} = Cdo\_1n_{clr} - Cdo$ which express the short time behavior of the pulse. We standardize $f2_{clr}$ and get f_norm = $(f2_{clr} - \min(f2_{clr})) / \max(f2_{clr} - \min(f2_{clr}))$. Again this procedure enables us to utilize the data independently of the curve form, potential and the step size. An example of the f_norm curve is shown in figure 7.

The next step is to make a least mean square line fit to ln(f_norm) in the range 0.2 < f_norm < 0.8, i.e. the initial part of the curve (different ranges might be used e.g. 0,1:0,9). An estimation of f_norm, f_norm_est, is then given by f_norm_est = $\exp(c_{clr}(3)^*t + c_{clr}(4))$ where $c_{clr}(3)$ and $c_{clr}(4)$ are the coefficients generated by the line fit.

By having performed the selection of data as described above, we know that f_norm = 1 corresponds to Cdo = $Cdo_{pre}$. This means that when f_norm_est equals the value 1 we are at what we define as $t0_{Cdo}$. $t0_{Cdo}$ is therefore easily calculated as $t0_{Cdo} = -c_{clr}(4)/c_{clr}(3)$.

We have now got the needed t0 values and can start adjusting the data. We start by synchronizing the two curves (i.e. moving the Cdo data so that $t0_{Cdo}$ coincides with $t0_{cdi}$).

The mean value of Cdi during the step is calculated. To get the most appropriate mean value we use data between $(t0_{Cdi} + 3$ minutes) and $(tf_{Cdi} - 1$ minute). This excludes any overshoot effects and possible end effects and focuses on data being the base for the clearance and access flow estimations. The mean value is denoted $Cdi_{step,mean}$.

Also a value representing the pre step Cdo parameter is needed. We will here use a mean value of the data. It is denoted $Cdo_{pre,mean}$.

We are interested mainly in the big variations in Cdi and will therefore filter the signal quite hard. Only data between t = $(t0_{Cdi} + 0.5$ minutes) and t = $(tf_{Cdi} - 0.5$ minutes) is filtered. The filter used is an exponential one, $Cdi_{filt,i} = (N-1)/N^* Cdi_{filt,i-1} + 1/N^*Cdi_{i-1}$. The filter factor N used for the used data is between 150 and 250 and in detail 200. The mean sampling interval for these data is between 0,01 and 0,1 and in detail 0.033 minutes, which corresponds to a time constant of about 6.6 minutes. As start value for the filter we use $Cdi_{step,set}$. The difference $Cdi_{diff} = Cdi_{filt} - Cdi_{step,mean}$

is created. A variation ($\Delta$Cdi) around the mean value is believed to give raise to a corresponding variation in Cdo ($\Delta$Cdo), see the simplified case shown in Figure 8. Such a variation in Cdi can be seen as a small step in the inlet conductivity and shall, governed by the clearance, give a corresponding variation in Cdo. We assume therefore that the following is true: (raise in Cdo (Cdo2-Cdo1) due to raise in Cdi) / (raise in Cdi (Cdi2-Cdi1)) = (variation in Cdo ($\Delta$Cdo)) / (variations in Cdi ($\Delta$Cdi)). This gives us the corrected Cdo values as: $Cdo_{corr}$ = Cdo + (Cdo2 - $Cdo_{pre,mean}$)* $Cdi_{diff}$ /step size. Here we encounter a small problem. The value of Cdo2 will not be known until we are ready to calculate the clearance. Therefore we use an estimate of it, called $Cdo2_{est}$, which is the value that the above assessed function Cdo_1n$_{clr}$ gives at t = t0$_{Cdo}$. Another problem is that the correction is actually only valid when the needles are in the normal position. In the reverse position the clearance is lower and the correction should hence have been somewhat smaller. Since the difference between $K_n$ and $K_r$ is quite small and since we are discussing only the correction, which is small, we assume that the error we introduce is very small.

Now that we have adjusted the data the method further comprises a step of consisting in mathematical computation of the conductivity curve downstream from the filtration unit in order thereby to determine a characteristic time tStartCF beyond which the conductivity curve has stabilized after undergoing the effects of the imposed change in conductivity. Said characteristic time tStartCF is given by the sum of two terms, a first term $t_{target}$ which is function of the conductivity curve and a second term tcbf which is function of the blood flow.

In determination of the first term $t_{target}$ for calculating the characteristic time we first estimate an intermediate time tA$_{clr}$ and we then derive the first term $t_{target}$ as following explained.

We will firstly obtain what we call tA$_{clr}$ which is a time representative of the effects of the transient due the conductivity change in the upstream liquid. Indeed tA$_{clr}$ depends upon several factors among which we can mention the filter volume, blood and dyalisis liquid flows, the conductivity step and so on depending on which tA$_{clr}$ moves near or far from the terminal instant of the transient. For obtaining tA$_{clr}$ we use $Cdo_{corr}$ data between t0$_{Cdo}$ and tRev. The procedure is similar to what was described for finding t0$_{Cdi}$ but instead of trying to find a "baseline" towards which the area under $Cdo_{corr}$ should be created we instead use the function unit_f above. The area referred to is therefore the one restrained by unit_f and the t-axis. We make a least mean square fit to the area data in the interval t = (tRev - 1.5 minutes) and t = tRev. tA$_{clr}$ is then defined as the point where the line crosses the t-axis, i.e. where the line has its zero value. Since the area function is nice, i.e. very little noise, one could (to get less calculations) instead of making a least mean square fit, use a line going through the endpoints of the interval.

The next step is to construct a line going through the points (t0$_{Cdo}$, CdoO) and (tA$_{clr}$, CdoA), see figure 2. This line is extrapolated to a conductivity equal to $Cdo_{pre}$+ step size. The time point then obtained is called $t_{target}$. Where, in time, this point is located, compared to t0$_{Cdo}$. is dependent on the shape of the curve, i.e. the dialyser used, blood flow, dialysis solution flow and also parameters origin from the patient. It expresses a sort of time constant for the curve.

To $t_{target}$ we will add a time, dependent on the blood flow (tcbf = 16/60 +260/Qb). The reason is that it is shown by the modeling work that the concentration in the body, Cvv (venous blood concentration), develops with a time delay of this size. This time is, since it is based on the blood flow, also linked to the shape of the Cdo curve. In the modeling work, the time used was about 1 minute. Also for clinically recorded data 1 minute was a good choice.

The time tcbf has two uses. The first is adding it to $t_{target}$. By that, we will get to a position on the Cdo curve where most of the initial effects have decayed. The time point obtained (tstartCF) is the one from which we start using Cdo data for the subsequent estimation. The second use is for finding the best time for the clearance calculation (see below).

We choose to make the line fit to data between t = tstartCF$_{clr}$ and t = (tstartCF$_{clr}$ + 1.5 minutes). We assume that, by using a fixed range relative to tstartCF$_{clr}$, we are using data from the same part of the curve independently of whatever parameters having influenced the curve. The line fit is the same as described above, i.e. we create the function f = ln(sign*(Cdi$_{step,mean}$ -Cdo$_{corr}$)) and make a least mean square fit to it. Observe that the Cdi used is now the step mean value of Cdi. As before we create Cdo_1n$_{clr}$ = Cdi$_{step,mean}$ - sign*exp(c(1)*t+c(2)), where c(1) and c(2) are the coefficients from the fit. The function Cdo_1n$_{clr}$ will then give us the sought Cdo2 needed for the clearance calculation.

Should it not be possible to use data up to t = (tstartCF$_{clr}$ + 1.5 minutes) for the curve fitting, i.e. tRev is passed, the rise is considered to slow and no calculation should be performed. The operator should be informed about it and what actions to take.

The clearance (effective) is calculated through the expression:

$$K_e = (Q_d + Q_{UF}) \cdot (1 - \frac{Cdo2 - Cdo1}{Cdi2 - Cdi1})$$

where Cdi2 is equal Cdi$_{step,mean}$ (alternatively also the set values Cdi$_{set,step}$ may be used) and Cdi1 is equal to Cdi$_{pre,mean}$ (alternatively also the set values Cdi$_{set,pre}$ may be used); it is also clear that, due to the symmetry of the clearance calculation expression, values of both Cdi2 and Cdi1, Cdo2 and Cdo1 may be inverted.

Cdo2 is the value that Cdo_1n$_{clr}$ gives at t = tCalc$_{clr}$ = tA$_{clr}$ + tcbf.

Cdo1 is given by function Cdo$_{pre}$, extended and taken at tCalc$_{clr}$. Cdo$_{pre}$ is created by performing a line fit to Cdo data in the same interval as is used when assessing Cdo$_{pre,mean}$. One may wonder why we are not creating and using an exponential function also for these data, but the reason is that it is difficult when Cdo and Cdi are close in value.

Qd and Quf are the flow rates of the dialysis solution and the UF respectively. Above is described the case where a fixed time of 1.5 minutes is used for fitting a curve to Cdo-data in order to get Cdo2. This gives, as an average, a better value on the effective clearance Ke. However, from a standard deviation of Ke point of view (if possible), it could perhaps be better to use as much data as possible from the step. If this is done we need however to move the calculation point somewhat towards the left since the whole curve and hence also Cdo2 otherwise becomes a bit too high.

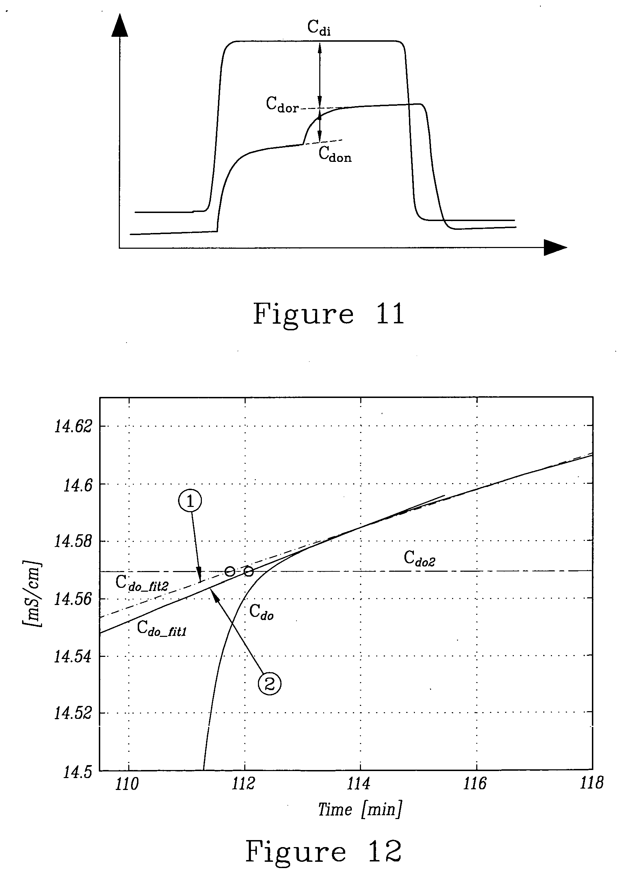

As shown in fig. 12 two line fits have been made to the Cdo curve, Cdo_fit1 and Cdo_fit2 when creating Cdo_fit1 we used data of an interval of 1,5 minutes; viceversa the line fit according to Cdo_fit2 required an interval of 5 minutes (i.e. more data have been used).

As can be seen the Cdo_fit2 curve is then lifted some (1) and hence giving somewhat higher conductivity values. Also the horizontal line Cdo2 has been drawn.

Assuming that we got the conductivity value Cdo2 from the first line fit at time t=112,1 min; to get the same Cdo2 value from the second line fit we need to move towards the left, to t=118 min.

In the real case, if, for stability reason, we would choose to use more data this would represent the second curve fit. Consequently we should move the calculation point to the left. One choice is to use t0$_{Cdo}$ ; this is of course not the proper point but it is one that is already in the algorithm.

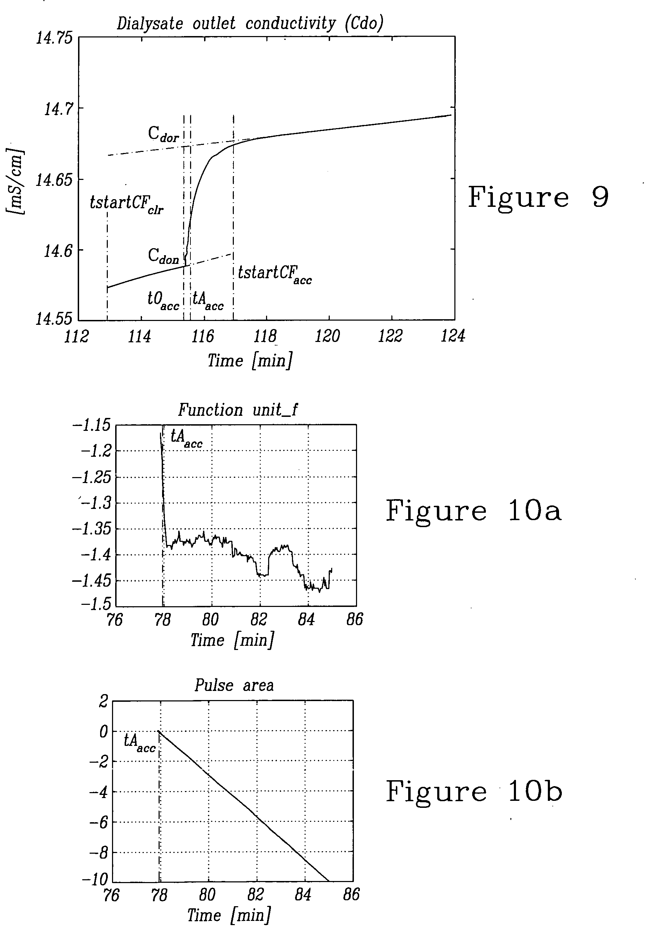

The access flow is calculated from the step one get when shifting the blood flow to the needles, i.e. blood taken out upstream is shifted with blood entering downstream. This creates a recirculation in the fistula, which affects the efficiency of the dialysis. K$_e$ becomes less. How much the flow reversal reduces the efficiency is dependent on the access flow. The effect of the flow reversal is seen in the Cdo-curve as a small step (Figure 9). The size of the step, along with the clearance gives the access flow.

The procedure for the access step calculations could theoretically be the same as for the clearance step. However, the noise is making this approach difficult since this step is much smaller than the clearance one. Therefore we need to do somewhat differently. We are going to describe two ways of finding the parameters needed. One that gives a more correct point for calculation of the access flow, but which might result in a larger variation. The second approach gives results which are the other way around.

The method for calculating fistula flow generally comprises the step of determining the filter clearance as above described, when reversing the blood flow direction to the fistula and determining, by means of mathematical calculation, an outlet conductivity prior the reversal of the blood flow Cdon; furthermore the method includes the step of determining a conductivity of the outlet process fluid Cdor following the reversal in blood flow and setting or estimating an inlet conductivity Cdi after the imposition of a change in conductivity.

If the switching of the blood flow to the fistula is made manually one should choose t0$_{acc}$ as the point in time at which the conductivities for the access flow calculation, should be taken. When shifting manually, an overshoot in C$_{do}$ is induced. Dependent on how the shift is done (e.g. how long it takes) the overshoot will vary from time to time. t0$_{acc}$ is independent of how the shifting is done. Therefore, the variations in Q$_a$ become less.

As in the clearance step, the correct time lies some time after t0$_{acc}$.

We want our method to find consistent values of t0$_{acc}$ independently of if the reversion is made manually or automatically. Since the Cdo curve, in these two cases, behave differently; the method of using the area does not lead to this consistency. Therefore, the route of using the "derivative" has been chosen.

One problem of using derivatives of signals is that they usually become noisy. The first action is therefore to filter Cdo$_{corr}$. The variable is called Cdo_flt. The same filter as described above is used. The difference is that the N is now 20 (with a sample time of 0.033 min this reflects a time constant of about 40 seconds).

We create the function f_flt = ln(sign*(Cdi$_{step,mean}$ - Cdo_flt)) and make a least mean square line fit to it between the time points t = (tfCdi- 4 minutes) and t = (tfCdi - 1 minute). This range represents a part of the curve where the transients have died out. The fit gives us an estimation of the of Cdo curve called Cdo_1n$_{acc,est}$(Cdo_1n$_{acc,est}$ = Cdi$_{step,mean}$ - sign*exp(c$_{acc,est}$(1)*t+c$_{acc,est}$(2)), c$_{acc,est}$ (1) and C$_{acc,est}$ (2) being the coefficients from the fit).

We now construct a function f2$_{acc}$ = Cdo_1n$_{acc,est}$ - Cdo_flt, which express the short time behavior of the pulse. Further we derive the difference function of f2 acc i.e. diff_f2$_{acc}$ = f2$_{acc}$(i)-f2$_{acc}$(i-1), i being the sample number. Standardizing diff_f2$_{acc}$ gives us the standardized derivative of f2$_{acc}$, f2$_{der\_norm}$ = diff_f2$_{acc}$ / min(diff_f2$_{acc}$). The function ranges from 0 to 1. In f2$_{der\_norm}$ we search for the point in time when the value for its first time becomes > 0.5 (called t0$_{acc,est}$). By having filtered the data, Cdo_flt is somewhat delayed compared to the original Cdo data. Therefore, we make an adjustment of t0$_{acc,est}$ and get t0$_{acc}$ = t0$_{acc,est}$ - 0.1 minute.

By using the f2$_{acc}$ function also on its way downwards one could get a better estimate of when the transients have died out. This means that we can find a good estimate of when to start using data for the line fit of Cdo$_{corr}$ during the reverse flow period. The point we are going to search for is the one obtained when f2$_{der\_norm}$, after having passed its maximum, gets below 0.2. This point in time plus a margin of 0.5 minutes gives us the time t = tstartCF$_{acc}$.

How the access flow is calculated is described below.

If the switching of the blood flow to the fistula is made automatically, a somewhat better point in time to use for the access flow calculation is tA$_{acc}$.

tA$_{gcc}$ is derived in the same way as was tA$_{clr}$. We create the function unit_f for data between t0$_{acc}$ and tf$_{Cdi}$ - 1 minute (unit_f$_{acc}$ = ln(sign * (Cdi$_{step,mean}$ - Cdo$_{corr}$)/potential)). We derive the area function. The area referred to is the one restrained by unit_f$_{acc}$ and the t-axis. We then make a least mean square fit to the area data in the interval t = tstartCF$_{acc}$ and t = tf$_{cdi}$ -1 minute. tA$_{acc}$ is then defined as the point where the line crosses the t-axis, i.e. where the line has its zero value.

In figure 10 are shown example plots of unit_f$_{acc}$ and the associated area.

The access flow is calculated using the expression

$$Q_{aw} = (K_e - Q_{UF}) \cdot (\frac{Cdor - Cdi}{Cdon - Cdor})$$

where K$_e$ is the effective clearance obtained as above. Q$_{uf}$ is the UF. The conductivities are best described by figures 9 and 11.

To get the values of the conductivities we create the function f in the same way as for the clearance step. We make a least mean square line fit to the function for data lying between t = tstartCF$_{acc}$ and t=tf$_{cdi}$ - 1 minute. The resulting Cdo estimation then becomes Cdo_1n$_{acc}$ = Cdi$_{step,mean}$ - sign*exp(c$_{acc}$(1)*t+c$_{acc}$ (2)), c$_{acc}$ (1) and c$_{acc}$ (2) being the coefficients from the fit. We do the same thing for the curve prior to the reversion. For this we use data between t = tstartCF$_{clr}$ and t = (t0$_{acc}$ - 0.5 minutes). As result we get Cdo_1n$_{pre,acc}$ = Cdi$_{step,mean}$ - sign*exp(c$_{pre,acc}$(1)*t+C$_{pre,acc}$ (2)).

Dependent on the situation of manual or automatic reversion of the blood flow, as described above, we use tA$_{acc}$ or t0$_{acc}$ as time point of when the conductivities are to be taken (= tCalc$_{acc}$). Cdo_1n$_{pre,acc}$ gives, at t = tCalc$_{acc}$, Cdon. Cdo_1n$_{acc}$ gives at the same time point Cdor. Cdi is given by Cdi$_{step,mean}$.

The access flow that has now been calculated is the blood water access flow Qaw. What we want is the whole blood access flow. With help from the book "Replacement of renal function by dialysis", fourth edition, chapter 2, page 41 one can set up the following expression for the conversion:

$$Q_a = \frac{Q_{aw}}{\left[(1 - \frac{Hct}{100}) * (1 - \frac{Tp}{1000}) + \frac{Hct}{100} * Fr\right]}$$

were Hct is the hematocrit, Tp is the total plasma protein content and Fr is the red cell water fraction (volume water in red cells / total volume of red cells). If we use average values for the different quantities (Hct = 35 %, Tp = 70g/l and Fr = 0.72) the expression becomes

$$Q_a = 1.168 * Q_{aw}$$

The invention achieves important advantages.

First of all it is to be noted that the method for determining the conductivity carried out in the machine according to the present invention allows to increase the accuracy of calculation of clearance and of access flow.

The method is adapted to be used with different blood treatment machines and gives good results for different patients in different conditions.

In other words the method is general.

The present method allows also to more accurately access a conductivity needed for calculation of the clearance and of access flow by giving better estimation of the time point when the transition effect starts and ends.

Finally the conductivity detections are as much as possible independent from the step size, the operating conditions and from the presence of undesired disturbances or noises.

**Claims**

1. Blood treatment machine comprising:

    - at least a filtration unit with a first compartment for the circulation of blood and a second compartment for the circulation of the treatment fluid, said first and second compartments being separated by interposing at least a semi-permeable membrane;
    - means for changing the conductivity of the treatment fluid upstream from the filtration unit;
    - at least a first and second sensor installed upstream and downstream from the filtration unit for measuring, respectively, the conductivity of the process fluid upstream and downstream from the unit;
    - a control unit governing said devices in order to change the conductivity of the process fluid and which is able to receive conductivity signals from the aforementioned first and second sensors, wherein the control unit carries out a method of determining conductivity of a treatment fluid downstream from the filtration unit, said method comprising the steps of:
    - creating a flow of treatment fluid through the second compartment of the filtration unit;
    - imposing, for a predetermined time interval, a change in the conductivity of the treatment fluid at the inlet of the filtration unit in order thereby to cause an induced conductivity change in the fluid at the outlet of said filtration unit;
    - measuring over time a predetermined number of conductivity values Cdo downstream from the filtration unit and belonging to a conductivity curve of the treatment fluid downstream from the filtration unit,

    **characterized in that** the method further comprises the steps of:

    - defining at least one interpolating mathematical function for the purpose of estimating the pattern of the conductivity curve Cdo downstream from the filtration unit in an interval of time after the occurrence of the induced conductivity change;
    - determining a characteristic measuring time $tcalc_{clr}$; the determination of the characteristic measuring time $tcalc_{clr}$ comprises the following sub steps:

        - estimation of an intermediate time $tA_{clr}$ representative of a transient due to the induced conductivity change in the fluid;
        - correction of the intermediate time $tA_{clr}$ through the addition of a second time term tcbf, which is a function of blood flow;

    - calculating the value of the interpolating mathematical function at said characteristic measuring time $tcalc_{clr}$, said value representing the conductivity value Cdo2 of the process fluid downstream from the filtration unit after the induced conductivity change.

2. Machine according to claim 1, **characterized in that** the conductivity change imposed in the inlet fluid is a known change in value remaining constant over time, said change particularly being a positive change in conductivity, i.e. an increase in conductivity.

3. Machine according to claim 2, **characterized in that** the characteristic measuring time $tcalc_{clr}$ is

$$tcalc_{clr} = tA_{clr} + tcbf$$

    where tcbf= 16/60 +260/Qb, where Qb=blood flow.

4. Machine according to claim 2, **characterized in that** the intermediate time $tA_{clr}$ is calculated using the function unit_f:

$$unit\_f = \ln(sign * (Cdi_{step,set} - Cdo)/potential);$$

    where potential $Cdi_{step,mean} - CdO_{pre,mean}$;
    where $Cdi_{step,set}$=value of the known change in conductivity at the filtration unit inlet;
    where Cdo= conductivity values measured downstream from the filtration unit;

where $Cdi_{step,mean}$ = inlet conductivity value after the change in conductivity
where $Cdo_{pre,mean}$ = average outlet conductivity value prior to the change in conductivity,
and by performing an interpolation with least squares estimation on the data of the area falling between said function and the x-coordinate time axis in a predetermined time interval, said intermediate time $tA_{clr}$ coinciding with the instant at which the interpolating function intersects the time axis, i.e. takes on a value of zero.

5. Machine according to claim 4, **characterized in that** the predetermined time interval for the least squares estimation of the function unit_f falls between (tRev-1.5 min) and tRev,
tRev being an instant of reversal of blood lines.

6. Machine according to claim 1, **characterized in that** it also comprises the following steps:

   - measuring over time a predetermined number of conductivity values of the treatment fluid upstream from the filtration unit and belonging to a conductivity curve of the treatment fluid upstream from the filtration unit;
   - determining a characteristic time $t0_{Cdo}$ of the conductivity curve of the treatment fluid downstream from the filtration unit, said characteristic time $t0_{Cdo}$ corresponding to the starting point of the conductivity curve of the treatment fluid downstream from the filtration unit;
   - determining a characteristic time $t0_{Cdi}$ of the conductivity curve of the treatment fluid upstream from the filtration unit, said characteristic time $t0_{Cdi}$ corresponding to the starting point of the conductivity curve of the treatment fluid upstream from the filtration unit;
   - synchronizing the conductivity curves on the basis of the characteristic times $t0_{Cdo}$ and $t0_{Cdi}$ determined by the curves upstream and downstream from the filtration unit to enable comparison of the respective conductivity values, said synchronizing being performed by moving one of the conductivity curve to the starting point of the other curve;
   - comparing the conductivity curves upstream and downstream from the filtration unit after they have been synchronized in order thereby to determine one or more downstream conductivity values.

7. Machine according to claim 6, **characterized in that** the characteristic time $t0_{Cdi}$ of the upstream conductivity curve coincides with the instant at which the change in conductivity occurs in the conductivity curve upstream from the filtration unit, the characteristic time $t0_{Cdo}$ of the downstream conductivity curve corresponding to the instant at which the induced conductivity change downstream from the filtration unit occurs.

8. Machine according to claim 7, **characterized in that** the characteristic time $t0_{Cdi}$ of the upstream conductivity curve is calculated by estimating an area defined below the inlet conductivity curve, the characteristic time $t0_{Cdi}$ coinciding with the instant at which the area under the curve takes on an average value greater than a predetermined threshold.

9. Machine according to claim 7, **characterized in that** the determination of the characteristic time $t0_{Cdo}$ of the conductivity curve downstream from the filtration unit comprises a step consisting in a preliminary estimation of the value of the characteristic time and the preliminary estimate is subsequently corrected.

10. Machine according to claim 9, **characterized in that** the preliminary estimation of the value of the characteristic time of the conductivity curve downstream from the filtration unit comprises the following sub steps:

    - determining the average conductivity at the outlet of the filtration unit $Cdo_{pre,mean}$ prior to the effects of the change in conductivity, said determination being made on the basis of an average, e.g. arithmetic mean, of the measured conductivity values Cdo prior to the effects of the change;
    - comparing the measured conductivity values at the outlet of the filtration unit Cdo with the previously determined average outlet conductivity value $Cdo_{pre,mean}$;
    - estimating the instant at which the measured conductivity values Cdo appear constantly greater than the previously calculated average outlet conductivity value $Cdo_{pre,mean}$.

11. Machine according to claim 10, **characterized in that** in the case where the measured conductivity values Cdo exceed the average outlet conductivity value $Cdo_{pre,mean}$ a number of times, the preliminary estimate of the characteristic time $t0_{Cdo}$ of the conductivity curve downstream from the filtration unit coincides with the instant of the last condition in which the measured conductivity values Cdo are greater than the average outlet conductivity value $Cdo_{pre,mean}$.

12. Machine according to claim 9, **characterized in that** the step of correcting the preliminary estimate of the characteristic time $t0_{Cdo}$ value of the conductivity curve downstream from the filtration unit comprises the following sub steps:

- creating an appropriate mathematical expression unit_f, which is a function of the conductivity values measured downstream from the filtration unit Cdo, and of the known change in the conductivity of the inlet fluid, $Cdi_{step,set}$, said expression being normalized if necessary;
- performing a least squares estimation of said expression unit_f in a predetermined time interval.

13. Machine according to claim 12, **characterized in that** the mathematical expression used is the following:

$$unit\_f = \ln(sign * (Cdi_{step,set} - Cdo)/potential))$$

where potential $Cdi_{step,mean} - CdO_{pre,mean}$;
where $Cdi_{step,set}$ = value of the known change in conductivity at the filtration unit inlet;
where $Cdo$ = conductivity values measured downstream from the filtration unit;
where $Cdi_{step,mean}$ = inlet conductivity value after the change in conductivity
where $Cdo_{pre,mean}$ = average outlet conductivity value prior to the change in conductivity.

14. Machine according to claim 13, **characterized in that** the least squares estimation is performed on the mathematical expression unit_f and the estimate is called

$$Cdo\_1n_{clr} = Cdi_{set,step} - sign*\exp(c_{clr}(1)*t + c_{clr}(2))$$

where $c_{clr}(1)$ $c_{clr}(2)$ are coefficients derived from the least squares interpolation of the mathematical expression unit_f.

15. Machine according to claim 12, **characterized in that** the step of correcting the preliminary estimate of the value of the characteristic time of the conductivity curve downstream from the filtration unit comprises the additional sub steps of:

- creating a mathematical function f_norm, which is a function of coefficients $c_{clr}(1)$ and $c_{clr}(2)$ derived from the previous least squares interpolation, a function of the known change in conductivity $Cdi_{step,set}$ of the inlet fluid and a function of the conductivity values downstream from the filtration unit Cdo, said mathematical function f_norm being normalized if necessary
- performing a least squares interpolation on the mathematical function f_norm within a predetermined interval of values.

16. Machine according to claims 14 and 15, **characterized in that** the mathematical function used is the following:

$$f\_norm = (f2_{clr} - \min(f2_{clr})) / \max(f2_{clr} - \min(f2_{clr}))$$

where $f2_{clr} = Cdo\_1n_{clr} - Cdo$

17. Machine according to claim 15, **characterized in that** it comprises an additional sub step consisting in the creation of a mathematical estimation function f_norm_est, which is a function of the coefficients $c_{clr}(3)$ $c_{clr}(4)$ derived from the least squares interpolation of the mathematical function.

18. Machine according to claim 16, **characterized in that** the predetermined interval of the mathematical function f_norm in which the interpolation is to be performed falls between 0.1 and 0.9 and 0.2 and 0.8 in particular.

19. Machine according to claim 17, **characterized in that** the corrected value $t0_{Cdo}$ of the characteristic time of the conductivity curve downstream from the filtration unit coincides with the instant at which the mathematical estimation function f_norm_est takes on a value of one.

20. Machine according to claim 17, **characterized in that** the mathematical estimation function used is the following:

$$f\_norm\_est = \exp(c_{clr}(3){*}t+c_{clr}(4))$$

where $c_{clr}(3)$ and $c_{clr}(4)$ are the coefficients derived from the least squares interpolation of the natural logarithm of the mathematical function f_norm.

21. Machine according to claim 20, **characterized in that** the corrected value of the characteristic time $t0_{Cdo}$ is obtained using the following formula:

$$t0_{Cdo} = -c_{clr}(4)/c_{clr}(3)$$

22. Machine according to claim 12, **characterized in that** the predetermined time interval of the mathematical expression unit_f in which the interpolation is to be performed essentially falls between an instant of reversal in the blood line tRev-1.5 minutes and the instant of reversal in the blood line tRev.

23. Machine according to claim 6, **characterized in that** the step of synchronizing the conductivity curves entails a relative translation between the two, ascribed a value D, which is a function of the characteristic times $t0_{Cdi}$ and $t0_{Cdo}$ determined by the curves upstream and downstream from the filtration unit.

24. Machine according to any of the previous claims, **characterized in that** it comprises a step of determining average inlet conductivity after the imposed change in conductivity $Cdi_{step,mean}$, said determination being based on a average, e.g. arithmetic mean, of the measured conductivity values Cdi after the effects of the change.

25. Machine according to claim 24, **characterized in that** the conductivity values Cdi used to determine the average inlet conductivity after the change in conductivity $Cdi_{step,mean}$ fall again within the time interval between the instant at which the change in conductivity occurs in the conductivity curve upstream from the filtration unit, $t0_{Cdi}$+3 minutes, and the instant at which the effects of the change in conductivity cease in the same curve, $tf_{Cdi}$-1 minute.

26. Machine according to any of the previous claims, **characterized in that** an instant $tf_{Cdi}$, at which the effects of the change in conductivity cease in the conductivity curve upstream from the filtration unit, is calculated by estimating an area defined below the imposed conductivity curve, said instant $tf_{Cdi}$ being identified by the moment at which the area under the curve takes on an average value below a predetermined threshold.

27. Machine according to any of the previous claims, **characterized in that** the conductivity values measured upstream from the filtration unit Cdi are filtered to eliminate the long term fluctuations between such values.

28. Machine according to claim 27, **characterized in that** only the inlet conductivity values Cdi measured within a significant time interval are filtered, for example in the interval falling between the instant at which the change in conductivity occurs in the conductivity curve upstream from the filtration unit, $t0_{Cdi}$+0.5 minutes, and the instant at which the effects of the change in conductivity cease in the same curve, $tf_{Cdi}$ - 0.5 minutes.

29. Machine according to claim 27, **characterized in that** the mathematical filter used is of the exponential type.

30. Machine according to claim 29, **characterized in that**, after filtering, the conductivity curve upstream from the filtration unit fits the following expression:

$$Cdi_{filt,i} = (N{-}1)/N{*}\ Cdi_{filt,i{-}1} + 1/N{*}Cdi_{i{-}1}$$

where N=filtering factor.

31. Machine according to claim 30, **characterized in that** the filtering factor N used takes on values between 150 and 250, 200 in particular, where the sampling frequency used for the Cdi data ranges from 0.01 to 0.1 min, 0.033 min. in particular.

32. Machine according to any of the previous claims, **characterized in that** the measured values of the conductivity curve downstream from the filtration unit Cdo are corrected on the basis of the conductivity fluctuations generated in the measured values of the conductivity curve upstream from the filtration unit Cdi .

33. Machine according to claims 27 and 32, **characterized in that** the measured values of the downstream conductivity curve Cdo are corrected on the basis of the filtered conductivity values $Cdi_{filt}$ measured upstream.

34. Machine according to claims 30 and 32, **characterized in that** the corrected conductivity curve downstream from the filtration unit fits the expression:

$$Cdo_{corr} = Cdo + (Cdo2 - Cdo_{pre,mean})* Cdi_{diff} /step\ size$$

where $Cdi_{diff} = Cdi_{filt} - Cdi_{step,mean}$;

$$step\ size = Cdi_{pre,mean} - Cdi_{step,mean}$$

Cdo2=conductivity value downstream from the filtration unit after the effects of the change in conductivity

35. Machine according to claims 14 and 34, **characterized in that**, for the purpose of calculating $Cdo_{corr}$, a preliminary estimate value of Cdo2 is used, said estimate being defined by the value that the function $Cdo\_In_{clr}$ assumes at the instant $t=t0_{Cdo}$

36. Machine according to claim 1, **characterized in that** it further comprises a step consisting in the mathematical computation of the conductivity curve Cdo downstream from the filtration unit in order thereby to determine a characteristic time tStartCF, beyond which the conductivity curve has stabilized after undergoing the effects of the imposed change in conductivity.

37. Machine according to claim 36, **characterized in that** said characteristic time tStartCF is given by the sum of two terms, a first term $t_{target}$, which is a function of the conductivity curve, and a second term tcbf, which is a function of blood flow.

38. Machine according to claim 37, **characterized in that** the second term tcbf fits the following relation:

$$tcbf = 16/60 + 260/Qb$$

where Qb=blood flow.

39. Machine according to claims 6 and 37, **characterized in that** the determination of the first term $t_{target}$ for calculating the characteristic time entails the sub steps of:

- estimating an intermediate time $tA_{clr}$
- deriving the first term $t_{target}$, said first term $t_{target}$ coinciding with the time that makes a straight line passing through points $(t0_{Cdo}; CdoO)$ and $(tA_{clr}; Cd0A)$ take on the value $Cdo_{pre,mean}+$ step size

where Cdo0 and CdoA are the values taken on by curve Cdo at instants $t0_{Cdo}$ and $tA_{clr}$. where $Cdo_{pre,mean}$= average outlet conductivity prior to the effects of the change in conductivity.

40. Machine according to claim 39, **characterized in that** the intermediate time $tA_{clr}$ is calculated using the function:

$$unit\_f = ln(sign * (Cdi_{step,set} - Cdo)/potential)$$

and performing an interpolation with a least squares estimation on the data of the area between said function and the time axis of the x-coordinates in a predetermined interval of time, said intermediate time $tA_{clr}$ coinciding with the instant at which the interpolating function intersects the time axis, i.e. takes on a value of zero.

**41.** Machine according to claim 40, **characterized in that** the predetermined time interval for the least squares estimation of the function unit_f falls between (tRev-1.5 min) and tRev.

**42.** Machine according to claim 1, **characterized in that** it further entails a step of calculating conductivity at the outlet of the filtration unit after the imposed change in conductivity.

**43.** Machine according to claim 42, **characterized in that** the step of calculating conductivity at the outlet of the filtration unit entails a sub step consisting in the least squares interpolation of a mathematical curve f, which is a function of $Cdi_{step,mean}$ and $Cdo_{corr}$, in a predetermined time interval.

**44.** Machine according to claim 43, **characterized in that** the mathematical curve takes on the following form:

$$f = \ln(sign*( Cdi_{step,mean} - Cdo_{corr}))$$

**45.** Machine according to claim 43, **characterized in that** said predetermined time interval falls between $tstartCF_{clr}$ and $tstartCF_{clr}$ +1.5 min.

**46.** Machine according to claim 43, **characterized in that** the step of calculating conductivity at the outlet of the filtration unit additionally comprises the sub step of creating an equation $Cdo\_1n_{clr}$, which is a function of $Cdi_{set,mean}$ and c(1) and c(2), where c(1) and c(2)=coefficients derived from the least squares interpolation of function f.

**47.** Machine according to claim 46, **characterized in that** the equation turns out to have the form:

$$Cdo\_1n_{clr} = Cdi_{set,mean} - sign*\exp(c(1)*t+c(2))$$

and the sought value Cdo2 is the value of the equation at $t=tCalc_{clr}$.

**Patentansprüche**

**1.** Blutbehandlungsmaschine umfassend:

- mindestens eine Filtrationseinheit mit einem ersten Abteil zur Blutzirkulation und einem zweiten Abteil zur Zirkulation des Behandlungsfluids, wobei die ersten und zweiten Abteile durch Zwischenlegen mindestens einer semipermeablen Membran voneinander getrennt sind;
- Mittel zum Ändern der Leitfähigkeit des Behandlungsfluids aufwärts von der Filtrationseinheit;
- mindestens einen ersten und einen zweiten Sensor, die aufwärts und abwärts von der Filtrationseinheit installiert sind, zum Messen der Leitfähigkeit des Prozessfluids aufwärts bzw. abwärts von der Einheit;
- eine Steuereinheit, die die Vorrichtungen regelt, um die Leitfähigkeit des Prozessfluids zu ändern, und die Leitfähigkeitssignale aus den vorgenannten ersten und zweiten Sensoren empfangen kann,

wobei die Steuereinheit ein Verfahren zum Bestimmen der Leitfähigkeit eines Behandlungsfluids abwärts von der Filtrationseinheit durchführt, wobei das Verfahren die folgenden Schritte umfasst:

- Erzeugen eines Flusses von Behandlungsfluid durch das zweite Abteil der Filtrationseinheit;
- Auferlegen, für eine vorbestimmte Zeitspanne, einer Änderung der Leitfähigkeit des Behandlungsfluids am Eingang der Filtrationseinheit, um dadurch eine veranlasste Leitfähigkeitsänderung im Fluid am Ausgang der Filtrationseinheit zu verursachen;
- Messen, über die Zeit, einer vorbestimmten Anzahl von Leitfähigkeitswerten Cdo abwärts von der Filtrationseinheit und einer Leitfähigkeitskurve des Behandlungsfluids abwärts von der Filtrationseinheit zugehörend, **dadurch gekennzeichnet, dass** das Verfahren weiter umfasst die Schritte von:
- Definieren mindestens einer mathematischen Interpolationsfunktion zum Zwecke des Schätzens des Musters der Leitfähigkeitskurve Cdo abwärts von der Filtrationseinheit in einer Zeitspanne nach dem Auftreten der veranlassten Leitfähigkeitsänderung;
- Bestimmen einer charakteristischen Messzeit $tcalc_{clr}$; wobei die Bestimmung der charakteristischen Messzeit tcalc$_{clr}$ die folgenden Unterschritte umfasst:

- Schätzen einer Zwischenzeit tA$_{clr}$, die einen Übergangsvorgang aufgrund der veranlassten Leitfähigkeitsänderung im Fluid darstellt;
- Korrigieren der Zwischenzeit tA$_{clr}$ durch die Ergänzung eines zweiten Zeitglieds tcbf, das eine Funktion des Blutflusses ist;

- Berechnen des Wertes der mathematischen Interpolationsfunktion zur charakteristischen Messzeit tcalc$_{clr}$, wobei der Wert den Leitfähigkeitswert Cdo2 des Prozessfluids abwärts von der Filtrationseinheit nach der veranlassten Leitfähigkeitsänderung darstellt.

2. Maschine nach Anspruch 1, **dadurch gekennzeichnet, dass** die im Eingangsfluid auferlegte Leitfähigkeitsänderung eine bekannte Wertänderung ist, die über die Zeit konstant bleibt, wobei die Änderung insbesondere eine positive Leitfähigkeitsänderung ist, d.h. eine Leitfähigkeitszunahme.

3. Maschine nach Anspruch 2, **dadurch gekennzeichnet, dass** die charakteristische Messzeit tcalc$_{clr}$

$$tcalc_{clr} = tA_{clr} + tcbf \quad \text{ist,}$$

ist, wobei tcbf = 16/60 + 260/Qb,
wobei Qb = Blutfluss.

4. Maschine nach Anspruch 2, **dadurch gekennzeichnet, dass** die Zwischenzeit tA$_{clr}$ unter Verwendung der Funktion unit_f berechnet wird:

$$unit\_f = \ln(sign * (Cdi_{step,set} - Cdo)/potential);$$

wobei potential = Cdi$_{step,mean}$ - Cdo$_{pre,mean}$;
wobei Cdi$_{step,set}$ = Wert der bekannten Leitfähigkeitsänderung am Eingang der Filtrationseinheit;
wobei Cdo = Leitfähigkeitswerte, die abwärts von der Filtrationseinheit gemessen werden;
wobei Cdi$_{step,mean}$ = Eingangsleitfähigkeitswert nach der Leitfähigkeitsänderung
wobei Cdo$_{pre,mean}$ = durchschnittlicher Ausgangsleitfähigkeitswert vor der Leitfähigkeitsänderung,
und durch Ausführung einer Interpolation mit Schätzung nach kleinsten Quadraten auf den Daten des Bereichs, der zwischen die Funktion und die Zeitachse der x-Koordinate in eine vorbestimmte Zeitspanne fällt, wobei die Zwischenzeit tA$_{clr}$ mit dem Punkt zusammenfällt, an dem die Interpolationsfunktion die Zeitachse durchschneidet, d.h. einen Wert von Null annimmt,.

5. Maschine nach Anspruch 4, **dadurch gekennzeichnet, dass** die vorbestimmte Zeitspanne für die Schätzung nach kleinsten Quadraten der Funktion unit_f zwischen (tRev - 1,5 min) und tRev liegt, wobei tRev ein Punkt der Umkehr der Blutleitungen ist.

6. Maschine nach Anspruch 1, **dadurch gekennzeichnet, dass** sie auch die folgenden Schritte umfasst:

- Messen, über die Zeit, einer vorbestimmten Anzahl von Leitfähigkeitswerten des Behandlungsfluids aufwärts von der Filtrationseinheit und einer Leitfähigkeitskurve des Behandlungsfluids aufwärts von der Filtrationseinheit zugehörend;
- Bestimmen einer charakteristischen Zeit t0$_{Cdo}$ der Leitfähigkeitskurve des Behandlungsfluids abwärts von der Filtrationseinheit, wobei die charakteristische Zeit t0$_{Cdo}$ dem Startpunkt der Leitfähigkeitskurve des Behandlungsfluids abwärts von der Filtrationseinheit entspricht;
- Bestimmen einer charakteristischen Zeit tO$_{Cdi}$ der Leitfähigkeitskurve des Behandlungsfluids aufwärts von der Filtrationseinheit, wobei die charakteristische Zeit t0$_{Cdi}$ dem Startpunkt der Leitfähigkeitskurve des Behandlungsfluids aufwärts von der Filtrationseinheit entspricht;
- Synchronisieren der Leitfähigkeitskurven auf Basis der charakteristischen Zeiten t0$_{Cdo}$ und t0$_{Cdi}$, die von der Kurven aufwärts und abwärts von der Filtrationseinheit bestimmt sind, um den Vergleich der jeweiligen Leitfähigkeitswerte zu ermöglichen, wobei die Synchronisation durch Bewegen einer der Leitfähigkeitskurven zum Startpunkt der anderen Kurve ausgeführt wird;
- Vergleichen der Leitfähigkeitskurven aufwärts und abwärts von der Filtrationseinheit, nachdem sie synchronisiert worden sind, um dadurch einen oder mehrere abwärtsliegende Leitfähigkeitswerte zu bestimmen.

7. Maschine nach Anspruch 6, **dadurch gekennzeichnet, dass** die charakteristische Zeit $t0_{Cdi}$ der aufwärtsliegenden Leitfähigkeitskurve mit dem Punkt, an dem die Leitfähigkeitsänderung in der Leitfähigkeitskurve aufwärts von der Filtrationseinheit auftritt, zusammenfällt, wobei die charakteristische Zeit $t0_{Cdo}$ der abwärtsliegenden Leitfähigkeitskurve dem Punkt, an dem die veranlasste Leitfähigkeitsänderung abwärts von der Filtrationseinheit auftritt, entspricht.

8. Maschine nach Anspruch 7, **dadurch gekennzeichnet, dass** die charakteristische Zeit $t0_{Cdi}$ der aufwärtsliegenden Leitfähigkeitskurve durch Schätzung eines Bereichs, der unter der Eingangsleitfähigkeitskurve definiert ist, berechnet wird, wobei die charakteristische Zeit $t0_{Cdi}$ mit dem Punkt, an dem der Bereich unter der Kurve einen größeren durchschnittlichen Wert als eine vorbestimmte Schwelle annimmt, zusammenfällt.

9. Maschine nach Anspruch 7, **dadurch gekennzeichnet, dass** die Bestimmung der charakteristischen Zeit $t0_{Cdo}$ der Leitfähigkeitskurve abwärts von der Filtrationseinheit einen Schritt bestehend aus einer vorläufigen Schätzung des Wertes der charakteristischen Zeit umfasst und danach die vorläufige Schätzung korrigiert wird.

10. Maschine nach Anspruch 9, **dadurch gekennzeichnet, dass** die vorläufige Schätzung des Wertes der charakteristischen Zeit der Leitfähigkeitskurve abwärts von der Filtrationseinheit die folgenden Unterschritte umfasst:

  - Bestimmen der durchschnittlichen Leitfähigkeit am Ausgang der Filtrationseinheit $Cdo_{pre,mean}$ vor den Auswirkungen der Leitfähigkeitsänderung, wobei die Bestimmung auf Basis eines Mittelwertes, z.B. eines arithmetischen Mittels, der gemessenen Leitfähigkeitswerte Cdo vor den Änderungsauswirkungen ausgeführt wird;
  - Vergleichen der gemessenen Leitfähigkeitswerte am Ausgang der Filtrationseinheit Cdo mit dem vorher bestimmten durchschnittlichen Ausgangsleitfähigkeitswert $Cdo_{pre,mean}$;
  - Schätzen des Punkts, an dem die gemessenen Leitfähigkeitswerte Cdo stetig größer zu sein scheinen als der vorher bestimmte durchschnittliche Ausgangsleitfähigkeitswert $Cdo_{pre,mean}$.

11. Maschine nach Anspruch 10, **dadurch gekennzeichnet, dass**, wenn die gemessenen Leitfähigkeitswerte Cdo den durchschnittlichen Ausgangsleitfähigkeitswert $Cdo_{pre,mean}$ mehrfach übersteigen, die vorläufige Schätzung der charakteristischen Zeit $t0_{Cdo}$ der Leitfähigkeitskurve abwärts von der Filtrationseinheit mit dem Punkt der letzten Bedingung zusammenfällt, in der die gemessenen Leitfähigkeitswerte Cdo größer sind, als der durchschnittliche Ausgangsleitfähigkeitswert $Cdo_{pre,mean}$.

12. Maschine nach Anspruch 9, **dadurch gekennzeichnet, dass** der Schritt des Korrigierens der vorläufigen Schätzung des Wertes der charakteristischen Zeit $t0_{Cdo}$ der Leitfähigkeitskurve abwärts von der Filtrationseinheit die folgenden Unterschritte umfasst:

  - Erstellen eines geeigneten mathematischen Ausdrucks unit_f, der eine Funktion der abwärts von der Filtrationseinheit Cdo gemessenen Leitfähigkeitswerte und der bekannten Leitfähigkeitsänderung des Eingangsfluids, $Cdi_{step,set}$, ist, wobei der Ausdruck, falls notwendig, normalisiert ist;

  - Ausführen einer Schätzung nach kleinsten Quadraten des Ausdrucks unit_f in einer vorbestimmten Zeitspanne.

13. Maschine nach Anspruch 12, **dadurch gekennzeichnet, dass** der verwendete mathematische Ausdruck wie folgt ist:

$$unit\_f = \ln(sign * (Cdi_{step,set} - Cdo)/potential))$$

wobei $potential = Cdi_{step,mean} - Cdo_{pre,mean}$;
wobei $Cdi_{step,set}$ = Wert der bekannten Leitfähigkeitsänderung am Eingang der Filtrationseinheit;
wobei $Cdo$ = Leitfähigkeitswerte, die abwärts von der Filtrationseinheit gemessen werden
wobei $Cdi_{step,mean}$ = Eingangsleitfähigkeitswert nach der Leitfähigkeitsänderung;
wobei $Cdo_{pre,mean}$ = durchschnittlicher Ausgangsleitfähigkeitswert vor der Leitfähigkeitsänderung.

**14.** Maschine nach Anspruch 13, **dadurch gekennzeichnet, dass** die Schätzung nach kleinsten Quadraten auf dem mathematischen Ausdruck unit_f ausgeführt wird, und die Schätzung ist wie folgt

$$Cdo\_1n_{clr} = Cdi_{set,step} - sign^*exp(c_{clr}(1)^*t + c_{clr}(2))$$

wobei $c_{clr}(1)$ und $c_{clr}(2)$ Koeffizienten aus der Interpolation nach kleinsten Quadraten des mathematischen Ausdrucks unit_f sind.

**15.** Maschine nach Anspruch 12, **dadurch gekennzeichnet, dass** der Schritt des Korrigierens der vorläufigen Schätzung des Wertes der charakteristischen Zeit der Leitfähigkeitskurve abwärts von der Filtrationseinheit die zusätzlichen Unterschritte umfasst, von:

- Erstellen einer mathematischen Funktion f_norm, die eine Funktion der Koeffizienten $c_{clr}(1)$ und $c_{clr}(2)$ aus der vorherigen Interpolation nach kleinsten Quadraten, eine Funktion der bekannten Leitfähigkeitsänderung $Cdi_{step,set}$ des Eingangsfluids und eine Funktion der Leitfähigkeitswerte abwärts von der Filtrationseinheit Cdo ist, wobei die mathematische Funktion f_norm, falls notwendig, normalisiert ist,
- Ausführen einer Interpolation nach kleinsten Quadraten auf der mathematischen Funktion f_norm in einem vorbestimmten Wertbereich.

**16.** Maschine nach den Ansprüchen 14 und 15, **dadurch gekennzeichnet, dass** die verwendete mathematische Funktion wie folgt ist:

$$f\_norm = (f2_{clr} - min(f2_{clr})) / max(f2_{clr} - min(f2_{clr})) \, ,$$

wobei $f2_{clr} = Cdo\_1n_{clr} - Cdo$.

**17.** Maschine nach Anspruch 15, **dadurch gekennzeichnet, dass** sie einen weiteren Unterschritt bestehend aus der Erstellung einer mathematischen Schätzfunktion f_norm_est umfasst, die eine Funktion der Koeffizienten $c_{clr}(3)$ und $c_{clr}(4)$ aus der Interpolation nach kleinsten Quadraten der mathematischen Funktion ist.

**18.** Maschine nach Anspruch 16, **dadurch gekennzeichnet, dass** die vorbestimmte Zeitspanne der mathematischen Funktion f_norm, in der die Interpolation auszuführen ist, zwischen 0,1 und 0,9, insbesondere zwischen 0,2 und 0,8, liegt.

**19.** Maschine nach Anspruch 17, **dadurch gekennzeichnet, dass** der korrigierte Wert $t0_{Cdo}$ der charakteristischen Zeit der Leitfähigkeitskurve abwärts von der Filtrationseinheit mit dem Punkt, an dem die mathematische Schätzfunktion f_norm_est einen Wert von eins annimmt, zusammenfällt.

**20.** Maschine nach Anspruch 17, **dadurch gekennzeichnet, dass** die verwendete mathematische Schätzfunktion wie folgt ist:

$$f\_norm\_est = exp(c_{clr}(3)^*t + c_{clr}(4)) \, ,$$

wobei $c_{clr}(3)$ und $c_{clr}(4)$ die Koeffizienten aus der Interpolation nach kleinsten Quadraten des natürliches Logarithmus der mathematischen Funktion f_norm sind.

**21.** Maschine nach Anspruch 20, **dadurch gekennzeichnet, dass** der korrigierte Wert der charakteristischen Zeit $t0_{Cdo}$ erhalten wird unter Verwendung der folgenden Formel:

$$t0_{Cdo} = -c_{clr}(4)/c_{clr}(3) \, .$$

**22.** Maschine nach Anspruch 12, **dadurch gekennzeichnet, dass** die vorbestimmte Zeitspanne des mathematischen Ausdrucks unit_f, in der die Interpolation auszuführen ist, wesentlich zwischen einem Punkt der Umkehr der Blutleitung tRev - 1,5 Minuten und dem Punkt der Umkehr der Blutleitung tRev liegt.

23. Maschine nach Anspruch 6, **dadurch gekennzeichnet, dass** der Schritt des Synchronisierens der Leitfähigkeitskurven eine relative Verschiebung zwischen den beiden beinhaltet, der ein Wert D zugeschrieben wird, der eine Funktion der charakteristischen Zeiten $t0_{Cdi}$ und $t0_{Cdo}$ ist, die von den Kurven aufwärts und abwärts von der Filtrationseinheit bestimmt sind.

24. Maschine nach einem der vorhergehenden Ansprüche, **dadurch gekennzeichnet, dass** sie einen Schritt des Bestimmens der durchschnittlichen Eingangsleitfähigkeit nach der auferlegten Leitfähigkeitsänderung $Cdi_{step,mean}$ umfasst, wobei die Bestimmung sich auf einen Mittelwert, z.B. ein arithmetisches Mittel, der gemessenen Leitfähigkeitswerte Cdi nach den Änderungsauswirkungen beruht.

25. Maschine nach Anspruch 24, **dadurch gekennzeichnet, dass** die zur Bestimmung der durchschnittlichen Eingangsleitfähigkeit nach der Leitfähigkeitsänderung $Cdi_{step,mean}$ verwendeten Leitfähigkeitswerte Cdi wieder in die Zeitspanne zwischen dem Punkt, an dem die Leitfähigkeitsänderung in der Leitfähigkeitskurve aufwärts von der Filtrationseinheit auftritt, $tO_{Cdi}$ + 3 Minuten, und dem Punkt, an dem die Auswirkungen der Leitfähigkeitsänderung in derselben Kurve beendet sind, $tf_{Cdi}$ - 1 Minute, fällt.

26. Maschine nach einem der vorhergehenden Ansprüche, **dadurch gekennzeichnet, dass** ein Punkt $tf_{Cdi}$, an dem die Auswirkungen der Leitfähigkeitsänderung in der Leitfähigkeitskurve aufwärts von der Filtrationseinheit beendet sind, durch Schätzung eines Bereichs unter der auferlegten Leitfähigkeitskurve berechnet wird, wobei der Punkt $tf_{Cdi}$ durch den Punkt, an dem der Bereich unter der Kurve einen durchschnittlichen Wert unter einer vorbestimmten Schwelle annimmt, definiert ist.

27. Maschine nach einem der vorhergehenden Ansprüche, **dadurch gekennzeichnet, dass** die aufwärts von der Filtrationseinheit gemessenen Leitfähigkeitswerte Cdi gefiltert werden, um die langfristigen Schwankungen zwischen den Werten zu beseitigen.

28. Maschine nach Anspruch 27, **dadurch gekennzeichnet, dass** nur die in einer signifikanten Zeitspanne gemessenen Eingangsleitfähigkeitswerte Cdi gefiltert werden, z.B. in der Zeitspanne zwischen dem Punkt, an dem die Leitfähigkeitsänderung in der Leitfähigkeitskurve aufwärts von der Filtrationseinheit auftritt, $t0_{Cdi}$ + 0,5 Minuten, und dem Punkt, an dem die Auswirkungen der Leitfähigkeitsänderung in derselben Kurve beendet sind, $tf_{Cdi}$ - 0,5 Minuten.

29. Maschine nach Anspruch 27, **dadurch gekennzeichnet, dass** der verwendete mathematische Filter exponentiellen Typs ist.

30. Maschine nach Anspruch 29, **dadurch gekennzeichnet, dass** nach der Filtration die Leitfähigkeitskurve aufwärts von der Filtrationseinheit Cdi den folgenden Ausdruck erfüllt:

$$Cdi_{filt,i} = (N-1)/N * Cdi_{filt,i-1} + 1/N * Cdi_{i-1}$$

wobei N = Filtrationsfaktor.

31. Maschine nach Anspruch 30, **dadurch gekennzeichnet, dass** der verwendete Filtrationsfaktor N Werte zwischen 150 und 250, insbesondere von 200, annimmt, wobei die für die Cdi-Daten verwendete Abtastfrequenz 0,01 bis 0,1 min, insbesondere 0,033 beträgt.

32. Maschine nach einem der vorhergehenden Ansprüche, **dadurch gekennzeichnet, dass** die gemessenen Werte der Leitfähigkeitskurve abwärts von der Filtrationseinheit Cdo auf der Basis der Leitfähigkeitsschwankungen, die in den gemessenen Werten der Leitfähigkeitskurve aufwärts von der Filtrationseinheit Cdi erzeugt werden, korrigiert werden.

33. Maschine nach den Ansprüchen 27 und 32, **dadurch gekennzeichnet, dass** die gemessenen Werte der abwärtsliegenden Leitfähigkeitskurve Cdo auf der Basis der vorher gemessenen, gefilterten Werte $Cdi_{filt}$ korrigiert werden.

34. Maschine nach den Ansprüchen 30 und 32, **dadurch gekennzeichnet, dass** die korrigierte Leitfähigkeitskurve abwärts von der Filtrationseinheit den folgenden Ausdruck erfüllt:

$$Cdo_{corr} = Cdo + (Cdo2 - Cdo_{pre,mean}) *$$

$$Cdi_{diff}/Inkrementgröße$$

wobei $Cdi_{diff} = Cdi_{filt} - Cdi_{step,mean}$;
Inkrementgröße $= Cdi_{pre,mean} - Cdi_{step,mean}$
$Cdo2 =$ Leitfähigkeitswert abwärts von der Filtrationseinheit nach den Auswirkungen der Leitfähigkeitsänderung.

35. Maschine nach den Ansprüchen 14 und 34, **dadurch gekennzeichnet, dass** zum Berechnen von $Cdo_{corr}$ ein vorläufiger Schätzwert von $Cdo2$ verwendet wird, wobei die Schätzung durch den Wert, den die Funktion $Cdo\_In_{clr}$ am Punkt $t=t0_{Cdo}$ annimmt, definiert ist.

36. Maschine nach Anspruch 1, **dadurch gekennzeichnet, dass** sie einen Schritt weiter umfasst, der aus der mathematischen Berechnung der Leitfähigkeitskurve $Cdo$ abwärts von der Filtrationseinheit besteht, um dadurch eine charakteristische Zeit tStartCF zu bestimmen, über der die Leitfähigkeitskurve sich stabilisiert hat, nachdem sie den Auswirkungen der auferlegten Leitfähigkeitsänderung unterzogen wurde.

37. Maschine nach Anspruch 36, **dadurch gekennzeichnet, dass** die charakteristische Zeit tStartCF durch die Summe zwei Glieder erhalten wird, eines ersten Glieds $t_{target}$, das eine Funktion der Leitfähigkeitskurve ist, und eines zweiten Glieds tcbf, die eine Funktion des Blutflusses ist.

38. Maschine nach Anspruch 37, **dadurch gekennzeichnet, dass** das zweite Glied tcbf die folgende Relation erfüllt:

$$tcbf = 16/60 + 260/Qb$$

wobei $Qb =$ Blutfluss.

39. Maschine nach den Ansprüchen 6 und 37, **dadurch gekennzeichnet, dass** die Bestimmung des ersten Glieds $t_{target}$ zum Berechnen der charakteristischen Zeit die Schritte beinhaltet, von:

   - Schätzen einer Zwischenzeit $tA_{clr}$,
   - Ableiten des ersten Glieds $t_{target}$, wobei das erste Glied $t_{target}$ mit der Zeit, die eine gerade Linie durch die Punkte ($t0_{Cdo}$; CdoO) und C($tA_{clr}$; Cd0A) bildet, zusammenfällt und den Wert $Cdo_{pre,mean} +$ Inkrementgröße annimmt

wobei Cdo0 und CdoA die von der Kurve $Cdo$ bei den Punkten $t0_{Cdo}$ und $tA_{clr}$ angenommenen Werte sind, wobei $Cdo_{p-re,mean} =$ die durchschnittliche Ausgangsleitfähigkeit vor den Auswirkungen der Leitfähigkeitsänderung ist.

40. Maschine nach Anspruch 39, **dadurch gekennzeichnet, dass** die Zwischenzeit $tA_{clr}$ berechnet wird unter Verwendung der folgenden Funktion:

$$\text{unit\_f} = \ln(\text{sign} * (Cdi_{step,set} - Cdo)/\text{potential})$$

und durch Ausführung einer Interpolation mit einer Schätzung nach kleinsten Quadraten der Daten des Bereichs zwischen der Funktion und die Zeitachse der x-Koordinaten in einer bestimmten Zeitspanne, wobei die Zwischenzeit $tA_{clr}$ mit dem Punkt, an dem die Interpolationsfunktion die Zeitachse durchschneidet, d.h. einen Wert von Null annimmt, zusammenfällt.

41. Maschine nach Anspruch 40, **dadurch gekennzeichnet, dass** die vorbestimmte Zeitspanne für die Schätzung nach kleinsten Quadraten der Funktion unit_f zwischen (tRev - 1,5 min) und tRev liegt.

42. Maschine nach Anspruch 1, **dadurch gekennzeichnet, dass** sie weiter einen Schritt des Berechnens der Leitfähigkeit am Ausgang der Filtrationseinheit nach der auferlegten Leitfähigkeitsänderung umfasst.

**43.** Maschine nach Anspruch 42, **dadurch gekennzeichnet, dass** der Schritt des Berechnens der Leitfähigkeit am Ausgang der Filtrationseinheit einen Unterschritt beinhaltet, der aus der Interpolation nach kleinsten Quadraten einer mathematischen Kurve f besteht, die eine Funktion von $Cdi_{step,mean}$ und $Cdo_{corr}$ in einer bestimmten Zeitspanne ist.

**44.** Maschine nach Anspruch 43, **dadurch gekennzeichnet, dass** die mathematische Kurve die folgende Form annimmt:

$$f = \ln(sign^*(\ Cdi_{step,mean} - Cdo_{corr}))$$ .

**45.** Maschine nach Anspruch 43, **dadurch gekennzeichnet, dass** die vorbestimmte Zeitspanne zwischen $tstartCF_{clr}$ und $tstartCF_{clr} +1,5$ min liegt.

**46.** Maschine nach Anspruch 43, **dadurch gekennzeichnet, dass** der Schritt des Berechnens der Leitfähigkeit am Ausgang der Filtrationseinheit den Unterschritt des Erstellens einer Gleichung $Cdo\_1n_{clr}$ weiter umfasst, die eine Funktion von $Cdi_{set,mean}$ und $c(1)$ und $c(2)$, wobei $c(1)$ und $c(2)$ = Koeffizienten aus der Interpolation nach kleinsten Quadraten der Funktion f sind.

**47.** Maschine nach Anspruch 46, **dadurch gekennzeichnet, dass** die Gleichung die folgende Form zeigt:

$$Cdo\_1n_{clr} = Cdi_{set,mean} - sign^*exp(c(1)^*t+c(2))$$ ,

und der gesuchte Wert Cdo2 der Wert der Gleichung bei $t=tCalc_{clr}$ ist.

**Revendications**

**1.** Machine pour le traitement du sang comprenant :

- au moins une unité de filtration avec un premier compartiment pour la circulation du sang et un deuxième compartiment pour la circulation du fluide de traitement, lesdits premier et deuxième compartiments étant séparés l'un de l'autre par interposition d'au moins une membrane semi-perméable ;
- moyens pour varier la conductivité du fluide de traitement en amont de l'unité de filtration ;
- au moins un premier et un deuxième capteur installés en amont et en aval de l'unité de filtration pour mesurer, respectivement, la conductivité du fluide de procès en amont et en aval de l'unité ;
- une unité de commande commandant lesdits dispositif afin de varier la conductivité du fluide de procès et à même de recevoir des signaux de conductivité des susdits premier et deuxième capteurs, où l'unité de commande exécute une méthode pour déterminer la conductivité d'un fluide de traitement en aval de l'unité de filtration, ladite méthode comprenant les étapes de :
- créer un flux de fluide de traitement à travers le deuxième compartiment de l'unité de filtration ;
- imposer pour un intervalle de temps préétabli une variation de la conductivité du fluide de traitement à l'entrée de l'unité de filtration afin ainsi de causer une variation de conductivité induite dans le fluide à la sortie de ladite unité de filtration ;
- mesurer dans le temps un nombre préétabli de valeurs de conductivité Cdo en aval de l'unité de filtration et faisant partie d'une courbe de conductivité du fluide de traitement en aval de l'unité de filtration,

**caractérisée en ce que** la méthode comprend en outre les étapes suivantes :

- définir au moins une fonction mathématique d'interpolation afin d'estimer le modèle de la courbe de conductivité Cdo en aval de l'unité de filtration dans un intervalle de temps après l'évènement de la variation de conductivité induite ;
- déterminer un temps de mesure caractéristique $tcalc_{clr}$; la détermination du temps de mesure caractéristique $tcalc_{clr}$ comprend les sous-étapes suivantes :

- estimer un temps intermédiaire tA$_{clr}$ représentant un transitoire dû à la variation de conductivité induite dans le fluide ;
- corriger le temps intermédiaire tA$_{clr}$ à travers l'addition d'un deuxième terme de temps tcbf, qui est une fonction du flux du sang ;

- calculer la valeur de la fonction mathématique d'interpolation audit temps de mesure caractéristique tcalc$_{clr}$, ladite valeur représentant la valeur de conductivité Cdo2 du fluide de procès en aval de l'unité de filtration après la variation de conductivité induite.

2. Machine selon la revendication 1, **caractérisée en ce que** la variation de conductivité est une variation de valeur connue qui reste constante dans le temps, ladite variation étant en particulier une variation positive de la conductivité, c'est-à-dire une augmentation de la conductivité.

3. Machine selon la revendication 2, **caractérisée en ce que** le temps de mesure caractéristique tcalc$_{clr}$ est

$$tcalc_{clr} = tA_{clr} + tcbf ,$$

où tcbf = 16/60 + 260/Qb,
où Qb = flux du sang.

4. Machine selon la revendication 2, **caractérisée en ce que** le temps intermédiaire tA$_{clr}$ est calculé en utilisant la fonction unit_f :

$$unit\_f = \ln(sign * (Cdi_{step,set} - Cdo)/potential);$$

où potential = Cdi$_{step,mean}$ - Cdo$_{pre,mean}$;
où Cdi$_{step,set}$ = valeur de la variation de conductivité connue à l'entrée de l'unité de filtration ;
où Cdo = valeurs de conductivité mesurées en aval de l'unité de filtration ;
où Cdi$_{step,mean}$ = valeur de conductivité en entrée après la variation de conductivité
où Cdo$_{pre,mean}$ = valeur de conductivité en sortie moyenne avant la variation de conductivité,
et en exécutant une interpolation des moindres carrés sur les données de la zone entre ladite fonction et l'axe de temps de la coordonnée x dans un intervalle de temps préétabli, ledit temps intermédiaire tA$_{clr}$ correspondant à l'instant auquel la fonction d'interpolation croise l'axe du temps, c'est-à-dire prend une valeur de zéro.

5. Machine selon la revendication 4, **caractérisée en ce que** l'intervalle de temps préétabli pour l'estimation des moindres carrés de la fonction unit_f est compris entre (tRev - 1,5 min) et tRev,
tRev étant un instant d'inversion des conduits du sang.

6. Machine selon la revendication 1, **caractérisée en ce que** elle comprend aussi les étapes suivantes :

- mesurer dans le temps un nombre préétabli de valeurs de conductivité du fluide de traitement en amont de l'unité de filtration et faisant partie d'une courbe de conductivité du fluide de traitement en amont de l'unité de filtration ;
- déterminer un temps caractéristique t0$_{Cdo}$ de la courbe de conductivité en aval de l'unité de filtration, ledit temps caractéristique t0$_{Cdo}$ correspondant au point de début de la courbe de conductivité du fluide de traitement en aval de l'unité de filtration ;
- déterminer un temps caractéristique tO$_{Cdi}$ de la courbe de conductivité en amont de l'unité de filtration, ledit temps caractéristique tO$_{Cdi}$ correspondant au point de début de la courbe de conductivité du fluide de traitement en amont de l'unité de filtration ;
- synchroniser les courbes de conductivité sur la base des temps caractéristiques t0$_{Cdo}$ et tO$_{Cdi}$ déter-minés par les courbes en amont et en aval de l'unité de filtration pour permettre la comparaison des valeurs de conductivité respectives, ladite synchronisation étant exécutée en déplaçant l'une des courbes de conductivité vers le point de début de l'autre courbe ;
- comparer les courbes de conductivité en amont et en aval de l'unité de filtration après qu'elles ont été synchronisées afin de déterminer ainsi une ou plusieurs valeurs de conductivité en aval.

**7.** Machine selon la revendication 6, **caractérisée en ce que** le temps caractéristique $t0_{Cdi}$ de la courbe de conductivité en amont correspond à l'instant auquel a lieu la variation de conductivité dans la courbe de conductivité en amont de l'unité de filtration, le temps caractéristique $t0_{Cdo}$ de la courbe de conductivité en aval correspondant à l'instant auquel a lieu la variation de conductivité induite en aval de l'unité de filtration.

**8.** Machine selon la revendication 7, **caractérisée en ce que** le temps caractéristique $t0_{Cdi}$ de la courbe de conductivité en amont est calculé en estimant une zone définie au-dessous de la courbe de conductivité en entrée, le temps caractéristique $t0_{Cdi}$ correspondant à l'instant auquel la zone au-dessous de la courbe prend une valeur moyenne supérieure au seuil préétabli.

**9.** Machine selon la revendication 7, **caractérisée en ce que** la détermination du temps caractéristique $t0_{Cdo}$ de la courbe de conductivité en aval de l'unité de filtration comprend une étape consistant dans une estimation préliminaire de la valeur du temps caractéristique, et l'estimation préliminaire est ensuite corrigée.

**10.** Machine selon la revendication 9, **caractérisée en ce que** l'estimation préliminaire de la valeur du temps caractéristique de la courbe de conductivité en aval de l'unité de filtration comprend les sous-étapes suivantes :

- déterminer la conductivité moyenne à la sortie de l'unité de filtration $Cdo_{pre,mean}$ avant les effets de la variation de conductivité, ladite détermination étant conduite sur la base d'une moyenne, par exemple une moyenne arithmétique, des valeurs de conductivité mesurées Cdo avant les effets de la variation ;
- comparer les valeurs de conductivité mesurées à la sortie de l'unité de filtration Cdo avec la valeur de conductivité en sortie moyenne calculée préalablement $Cdo_{pre,mean}$ ;
- estimer l'instant auquel les valeurs de conductivité mesurées Cdo apparaissent constamment supérieures à la valeur de conductivité en sortie moyenne calculée préalablement $Cdo_{pre,mean}$.

**11.** Machine selon la revendication 10, **caractérisée en ce que** au cas où les valeurs de conductivité mesurées Cdo seraient supérieures à la valeur de conductivité en sortie moyenne $Cdo_{pre,mean}$ plusieurs fois, l'estimation préliminaire du temps caractéristique $t0_{Cdo}$ de la courbe de conductivité en aval de l'unité de filtration correspond à l'instant de la dernière condition dans laquelle les valeurs de conductivité mesurées Cdo sont supérieures à la valeur de conductivité en sortie moyenne $Cdo_{pre,mean}$.

**12.** Machine selon la revendication 9, **caractérisée en ce que** l'étape de corriger l'estimation préliminaire de la valeur du temps caractéristique $t0_{Cdo}$ de la courbe de conductivité en aval de l'unité de filtration comprend les sous-étapes suivantes :

- création d'une expression mathématique adaptée unit_f, qui est une fonction des valeurs de conductivité mesurées en aval de l'unité de filtration Cdo et de la variation de conductivité connue du fluide en entrée, $Cdi_{step,set}$, l'expression étant normalisée si nécessaire ;
- exécution d'une estimation des moindres carrés de ladite expression unit_f dans un intervalle de temps préétabli.

**13.** Machine selon la revendication 12, **caractérisée en ce que** l'expression mathématique utilisée est la suivante:

$$\text{unit\_f} = \ln(\text{sign} * (Cdi_{step,set} - Cdo)/\text{potential}))$$

où potential = $Cdi_{step,mean} - Cdo_{pre,mean}$ ;
où $Cdi_{step,set}$ = valeur de la variation de conductivité connue à l'entrée de l'unité de filtration ;
où Cdo = valeurs de conductivité mesurées en aval de l'unité de filtration ;
où $Cdi_{step,mean}$ = valeur de conductivité en entrée après la variation de conductivité ;
où $Cdo_{pre,mean}$ = valeur de conductivité en sortie moyenne avant la variation de conductivité.

**14.** Machine selon la revendication 13, **caractérisée en ce que** l'estimation des moindres carrés est exécutée sur l'expression mathématique unit_f, et l'estimation est

$$Cdo\_1n_{clr} = Cdi_{set,step} - \text{sign}*\exp(c_{clr}(1)*t + c_{clr}(2))$$

où $C_{clr}$ (1) et $C_{clr}$ (2) sont des coefficients dérivés de l'interpolation des moindres carrés de l'expression mathématique unit_f.

**15.** Machine selon la revendication 12, **caractérisée en ce que** l'étape de corriger l'estimation préliminaire de la valeur du temps caractéristique de la courbe de conductivité en aval de l'unité de filtration comprend les sous-étapes additionnelles de :

- créer une fonction mathématique f_norm, qui est une fonction des coefficients $C_{clr}$ (1) et $C_{clr}$ (2) dérivés de l'interpolation préalable des moindres carrés, une fonction de la variation de conductivité connue $Cdi_{step,set}$ du fluide en entrée et une fonction des valeurs de conductivité en aval de l'unité de filtration Cdo, ladite fonction mathématique f_norm étant normalisée si nécessaire,
- exécution d'une interpolation des moindres carrés sur la fonction mathématique f_norm dans un intervalle de valeurs préétabli.

**16.** Machine selon les revendications 14 et 15, **caractérisée en ce que** la fonction mathématique utilisée est la suivante:

$$f\_norm = (f2_{clr} - \min(f2_{clr})) / \max(f2_{clr} - \min(f2_{clr})) ,$$

où $f2_{clr} = Cdo\_1n_{clr} - Cdo$.

**17.** Machine selon la revendication 15, **caractérisée en ce que** elle comprend une sous-étape additionnelle consistant dans la création d'une fonction d'estimation mathématique f_norm_est, qui est une fonction des coefficients $C_{clr}$ (3) et $C_{clr}$ (4) dérivés de l'interpolation des moindres carrés de la fonction mathématique.

**18.** Machine selon la revendication 16, **caractérisée en ce que** l'intervalle préétabli de la fonction mathématique f_norm dans lequel l'interpolation doit être exécuté est compris entre 0,1 et 0,9, en particulier entre 0,2 et 0,8.

**19.** Machine selon la revendication 17, **caractérisée en ce que** la valeur corrigée $t0_{Cdo}$ du temps caractéristique de la courbe de conductivité en aval de l'unité de filtration correspond à l'instant auquel la fonction d'estimation mathématique f_norm_est prend une valeur de un.

**20.** Machine selon la revendication 17, **caractérisée en ce que** la fonction d'estimation mathématique utilisée est la suivante:

$$f\_norm\_est = \exp(c_{clr} (3)^* t + c_{clr} (4)) ,$$

où $C_{clr}$ (3) et $C_{clr}$ (4) sont les coefficients dérivés de l'interpolation des moindres carrés du logarithme naturel de la fonction mathématique f_norm.

**21.** Machine selon la revendication 20, **caractérisée en ce que** la valeur corrigée du temps caractéristique $t0_{Cdo}$ est obtenu en utilisant la formule suivante:

$$t0_{Cdo} = -c_{clr} (4)/c_{clr} (3) .$$

**22.** Machine selon la revendication 12, **caractérisée en ce que** l'intervalle de temps préétabli de l'expression mathématique unit_f dans lequel l'interpolation doit être exécutée est compris entre un instant d'inversion dans le conduit du sang tRev - 1,5 minutes et l'instant d'inversion dans le conduit du sang tRev.

**23.** Machine selon la revendication 6, **caractérisée en ce que** l'étape de synchroniser les courbes de conductivité inclut une translation relative entre les deux, à laquelle on assigne une valeur D, qui est une fonction des temps caractéristiques $t0_{Cdi}$ et $t0_{Cdo}$ déterminés par les courbes en amont et en aval de l'unité de filtration.

**24.** Machine selon l'une quelconque des revendications précédentes, **caractérisée en ce que** elle comprend une étape de déterminer la conductivité en entrée moyenne après la variation de conductivité imposée $Cdi_{step,mean}$, ladite détermination étant conduite sur la base d'une moyenne, par exemple une moyenne arithmétique, des valeurs de conductivité mesurées Cdi après les effets de la variation.

25. Machine selon la revendication 24, **caractérisée en ce que** les valeurs de conductivité Cdi utilisées pour déterminer la conductivité en entrée moyenne après la variation de conductivité $Cdi_{step,mean}$ se trouvent de nouveau dans l'intervalle de temps entre l'instant auquel la variation de conductivité a lieu dans la courbe de conductivité en amont de l'unité de filtration, $t0_{Cdi}$ + 3 minutes, et l'instant auquel les effets de la variation de conductivité se terminent dans la même courbe, $tf_{Cdi}$ - 1 minute.

26. Machine selon l'une quelconque des revendications précédentes, **caractérisée en ce que** un instant $tf_{Cdi}$ auquel les effets de la variation de conductivité se terminent dans la courbe de conductivité en amont de l'unité de filtration est calculé en estimant la zone définie au-dessous de la courbe de conductivité imposée, ledit instant $tf_{Cdi}$ étant défini par l'instant auquel la zone au-dessous de la courbe prend une valeur moyenne au-dessous d'un seuil préétabli.

27. Machine selon l'une quelconque des revendications précédentes, **caractérisée en ce que** les valeurs de conductivité mesurées en amont de l'unité de filtration Cdi sont filtrées pour éliminer les fluctuations de longue durée entre lesdites valeurs.

28. Machine selon la revendication 27, **caractérisée en ce que** seulement les valeurs de conductivité en entrée Cdi mesurées dans un intervalle de temps significatif sont filtrées, par exemple dans l'intervalle entre l'instant auquel la variation de conductivité a lieu dans la courbe de conductivité en amont de l'unité de filtration, $t0_{Cdi}$ + 0,5 minutes, et l'instant auquel les effets de la variation de conductivité se terminent dans la même courbe, $tf_{Cdi}$ - 0,5 minutes.

29. Machine selon la revendication 27, **caractérisée en ce que** le filtre mathématique utilisé est de type exponentiel.

30. Machine selon la revendication 29, **caractérisée en ce que** après la filtration la courbe de conductivité en amont de l'unité de filtration satisfait l'expression suivante:

$$Cdi_{filt,i} = (N-1)/N* Cdi_{filt,i-1} + 1/N*Cdi_{i-1}$$

où N = facteur de filtration.

31. Machine selon la revendication 30, **caractérisée en ce que** le facteur de filtration utilisé prend des valeurs entre N et 150 et 250, en particulier de 200, où la fréquence d'échantillonnage utilisée pour les données Cdi va de 0,01 à 0,1 min, en particulier 0,033.

32. Machine selon l'une quelconque des revendications précédentes, **caractérisée en ce que** les valeurs mesurées de la courbe de conductivité en aval de l'unité de filtration Cdo sont corrigées sur la base des fluctuations de conductivité générées dans les valeurs mesurées de la courbe de conductivité en amont de l'unité de filtration Cdi.

33. Machine selon les revendications 27 et 32, **caractérisée en ce que** les valeurs mesurées de la courbe de conductivité en aval Cdo sont corrigées sur la base des valeurs de conductivité filtrées $Cdi_{filt}$.

34. Machine selon les revendications 30 et 32, **caractérisée en ce que** la courbe de conductivité corrigée en aval de l'unité de filtration satisfait l'expression:

$$Cdo_{corr} = Cdo + (Cdo2 - Cdo_{pre,mean}) * Cdi_{diff}/dimension$$

$$de\ l'incrément$$

où $Cdi_{diff}$ = $Cdi_{filt}$ - $Cdi_{step,mean}$ ;
dimension de l'incrément = $Cdi_{pre,mean}$ - $Cdi_{step,mean}$ Cdo2 = valeur de conductivité en aval de l'unité de filtration après les effets de la variation de conductivité.

35. Machine selon les revendications 14 et 34, **caractérisée en ce que** pour calculer $Cdo_{corr}$ on utilise une valeur d'estimation préliminaire de Cdo2, ladite estimation étant défini par la valeur prise par la fonction $Cdo\_1n_{clr}$ à l'instant $t=t0_{Cdo}$.

36. Machine selon la revendication 1, **caractérisée en ce que** elle comprend en outre une étape consistant dans le calcul mathématique de la courbe de conductivité Cdo en aval de l'unité de filtration afin ainsi de déterminer un temps caractéristique tStartCF au-delà duquel la courbe de conductivité s'est stabilisée après avoir subi les effets de la variation de conductivité imposée.

37. Machine selon la revendication 36, **caractérisée en ce que** ledit temps caractéristique tStartCF est obtenu de la somme de deux éléments, un premier élément $t_{target}$ qui est une fonction de la courbe de conductivité, et un deuxième élément tcbf qui est une fonction du flux du sang.

38. Machine selon la revendication 37, **caractérisée en ce que** le deuxième élément tcbf satisfait la relation suivante :

$$tcbf = 16/60 + 260/Qb$$

où Qb = flux du sang.

39. Machine selon les revendications 6 et 37, **caractérisée en ce que** la détermination du premier élément $t_{target}$ pour calculer le temps caractéristique inclut les sous-étapes de:

- estimer un temps intermédiaire $tA_{clr}$,
- dériver le premier élément $t_{target}$, ledit premier élément $t_{target}$ correspondant au temps qui forme une ligne droite traversant les points $(t0_{Cdo}; CdoO)$ et $(tA_{clr}; Cd0A)$, et prend la valeur $Cdo_{pre,mean}$ + dimension de l'incrément

où Cdo0 et CdoA sont les valeurs prises par la courbe Cdo aux instants $t0_{Cdo}$ et $tA_{clr}$, où $Cdo_{pre,mean}$ = conductivité en sortie moyenne avant les effets de la variation de conductivité.

40. Machine selon la revendication 39, **caractérisée en ce que** le temps intermédiaire $tA_{clr}$ est calculé en utilisant la fonction:

$$unit\_f = \ln(sign * (Cdi_{step,set} - Cdo)/potential)$$

et en exécutant une interpolation avec une estimation des moindres carrés sur les données de la zone entre ladite fonction et l'axe du temps des coordonnées x dans un intervalle de temps préétabli, ledit temps intermédiaire $tA_{clr}$ correspondant à l'instant auquel la fonction d'interpolation croise l'axe du temps, c'est-à-dire prend une valeur de zéro.

41. Machine selon la revendication 40, **caractérisée en ce que** l'intervalle de temps préétabli pour l'estimation de moindres carrés de la fonction unit_f est compris entre (tRev - 1,5 min) et tRev.

42. Machine selon la revendication 1, **caractérisée en ce que** elle comprend en outre une étape de calculer la conductivité à la sortie de l'unité de filtration après la variation de conductivité imposée.

43. Machine selon la revendication 42, **caractérisée en ce que** l'étape de calculer la conductivité à la sortie de l'unité de filtration inclut une sous-étape consistant dans l'interpolation des moindres carrés d'une courbe mathématique f, qui est une fonction de $Cdi_{step,mean}$ et $Cdo_{corr}$ dans un intervalle de temps préétabli.

44. Machine selon la revendication 43, **caractérisée en ce que** la courbe mathématique prend la forme suivante:

$$f = \ln(sign*(Cdi_{step,mean} - Cdo_{corr}))$$

45. Machine selon la revendication 43, **caractérisée en ce que** ledit intervalle de temps préétabli est compris entre $tstartCF_{clr}$ et $tstartCF_{clr}$ + 1,5 min.

46. Machine selon la revendication 43, **caractérisée en ce que** l'étape de calculer la conductivité à la sortie de l'unité de filtration comprend en outre la sous-étape de créer une équation $Cdo\_1n_{clr}$ qui est une fonction de $Cdi_{set,mean}$ et $c(1)$ et $c(2)$, où $c(1)$ et $c(2)$ = coefficients dérivés de l'interpolation des moindres carrés de la fonction f.

47. Machine selon la revendication 46, **caractérisée en ce que** l'équation a la forme suivante:

$$Cdo\_1n_{clr} = Cdi_{set,mean} - sign \cdot exp(c(1) \cdot t + c(2)),$$

et la valeurs recherchée Cdo2 est la valeur de l'équation à $t = tCalc_{clr}$.

Figure 1

Figure 2

Figure 3

Figure 4

Figure 5

Figure 6

Functions f_norm and f_norm_est (logfile 03210901.log)

Time [min]

# Figure 7

# Figure 8

Dialysate outlet conductivity (Cdo)

Figure 9

Function unit_f

Figure 10a

Pulse area

Figure 10b

Figure 11

Figure 12

**EP 1 604 698 B1**

**Patent documents cited in the description**

- EP 547025 A
- EP 658352 A

- WO 03066135 A1